# EUROPEAN PATENT SPECIFICATION

(11) **EP 2 087 115 B1**
(45) Date of publication and mention of the grant of the patent: **21.11.2012**
(21) Application number: 07843386.9
(22) Date of filing: 27.09.2007
(51) Int. Cl.: C12N 15/113, A61K 31/7088, A61P 35/00

(54) **BLOCKING OF GENE EXPRESSION IN EUKARYOTIC CELLS**
BLOCKIERUNG VON GENEXPRESSIONEN IN EUKARYOTISCHEN ZELLEN
BLOCAGE D'UNE EXPRESSION GÉNIQUE DANS DES CELLULES EUKARIOTES

(30) Priority: 27.09.2006 US 847758 P
(43) Date of publication of application: 12.08.2009
(60) Divisional application: 12167692.8
(73) Proprietor: Novarx, San Diego CA 92121-2635 (US)
(72) Inventor: FAKHRAI, Habib, La Jolla CA 92037 (US); FAKHRAI, Farideh, La Jolla CA 92037 (US)
(74) Representative: Hatzmann, Martin
(86) International application number: PCT/US2007/079763
(87) International publication number: WO 2008/039937

(56) References cited:
- WO-A-2004/044245
- WO-A-2005/103254
- US-A1- 2006 099 619
- AGRAWAL, S. (ED.) - BRYSCH, W. ET AL.: "Antisense-mediated inhibition of protein synthesis" METHODS IN MOLECULAR MEDICINE: ANTISENSE THERAPEUTICS, 1997, pages 159-182, XP002482902 Humana PressInc.; Totowa, NJ
- WICKSTROM E ED - ERICKSON R P ET AL: "ANTISENSE DNA THERAPEUTICS: NEUTRAL ANALOGUES AND THEIR STEREOCHEMISTRY" GENE REGULATION. BIOLOGY OF ANTISENSE RNA AND DNA, NEW YORK, RAVEN PRESS, US, 1992, pages 119-132, XP002014193
- AMARZGUIOUI M ET AL: "An algorithm for selection of functional siRNA sequences" BIOCHEMICAL AND BIOPHYSICAL RESEARCH COMMUNICATIONS, vol. 316, no. 4, 16 April 2004 (2004-04-16), pages 1050-1058, XP002316492 ISSN: 0006-291X cited in the application
- VALENCIA-SANCHEZ MARCO ANTONIO ET AL: "Control of translation and mRNA degradation by miRNAs and siRNAs" GENES & DEVELOPMENT, vol. 20, no. 5, March 2006 (2006-03), pages 515-524, XP002482903 ISSN: 0890-9369
- SIOLAS D ET AL: "Synthetic shRNAs as potent RNAi triggers" NATURE BIOTECHNOLOGY, vol. 23, no. 2, 26 December 2004 (2004-12-26), pages 227-231, XP002399750 ISSN: 1087-0156 cited in the application

## Description

### Field of the Invention

The present invention relates to methods for reliably selecting and designing a sequence for efficient short interference RNA (siRNA) molecules. Additionally, it concerns antisense compositions and methods.

### Description of the Related Art

RNA interference has rapidly displaced antisense and ribozymes as the preferred means for sequence-specific gene inhibition in cell culture studies (Bantounas et al., 2004 J Mol Endocrinol 33:545-557; Lu et al., 2003 Curr Opin Mol Ther 5:225-234; McManus and Sharp, 2002 Nature Med 3:737-747). Along with rapid adoption as a tool for functional genomics, expanding studies on RNA interference (RNAi) itself have greatly enhanced our understanding of this endogenous gene inhibition process since discovery that posttranscriptional gene silencing (PTGS) in plants is also active in animal cells. A major challenge is the growing appreciation that the RNAi system is involved in several endogenous activities with differing roles.

One of the earliest recognized roles of RNAi is as an antiviral response triggered by the double-stranded (ds) RNA genome of the double-stranded RNA virus. For such a response to be effective, strong and selective gene inhibition is important. This gene inhibition function operates through an active intermediate that is short fragments of the dsRNA genome, now called short interfering RNA (siRNA). The RNAi antiviral response generally is accompanied by interferon and other events induced by recognition of the dsRNA viral genome. An important finding has been that introduction of siRNA with sequences matching endogenous genes, instead of invading virus genomes, leads to activation of the RNAi system and inhibition of that endogenous gene. Importantly, studies have shown that introduction of these artificial siRNA usually avoids the interferon and other concomitant biological responses (McManus and Sharp, 2002 Nature Med 3:737-747). Also, the RNAi machinery has been found to perform its function in the cytoplasmic compartment, reducing the hurdles for intracellular siRNA delivery compared to gene therapy requirements for delivery to the nucleus.

Another major RNAi role is the regulation of cellular activity by modulating endogenous gene expression. This function operates, at least in part, through an active intermediate that is a short expressed RNA with an imperfect palindrome sequence, now called micro interfering RNA (miRNA). The palindrome sequence forms an imperfect dsRNA segment with a loop of single-stranded (ss) RNA at one end, or a hairpin type of structure, mimicked by expressed short hairpin RNA (shRNA). This RNAi role regulating endogenous gene expression may help explain why the introduction of siRNA matching endogenous genes shows strong and selective inhibition of matching endogenous genes.

Thus while the specific role of RNAi can vary considerably, the fundamental activity of RNAi is blocking expression of specific genes. Consequently, it is not surprising that different RNAi processes have very similar mechanisms of action and share machinery. The shared machinery may aid efforts to harness RNAi for our own purposes by allowing greater utility of the techniques developed.

The term "antisense" originally referred to an oligonucleotide sequence designed to complement a pre-mRNA or mRNA, both of which are "sense" molecules, so that the ultimate translation of that RNA would be inhibited.

Theoretically, when the two molecules were in close proximity, they would hybridize to each other and the target RNA would therefore no longer be accessible to translation initiation factors and ribosomes. In effect, the gene coding for the target RNA would be "turned off." This definition of antisense now includes oligonucleotides ("ON"; short RNA molecules) and oligodeoxynucleotides ("ODN"; short, single stranded DNA molecules) that bind double stranded DNA or areas of double stranded secondary structure in RNA to form triplexes. Indeed, the current definition of antisense covers the entire range of ON/ODN/nucleic acid interaction.

In the mid 1970s, some initial research was done using antisense compounds, but the idea of using complementary nucleic acid sequences to affect DNA or RNA function was not seriously pursued until the mid to late 1980s. At that time two developments made widespread antisense research possible: (1) protein and DNA sequencing technology and (2) nucleic acid synthesizing technology. Now the sequences of target RNAs could be determined and antisense molecules against these RNAs could be synthesized. Since then, research using antisense strategies has greatly increased.

Historically antisense has been used in two main areas of research: loss-of function studies and therapeutic use. In loss-of function studies, a gene or its mRNA is "turned off" by complementary antisense and the resulting phenotype noted. RNA and DNA antisense have been used to both control and study the functions of many diverse genes in a variety of prokaryotic and eukaryotic organisms. Technology involving the development of antisense therapeutics is also showing rapid growth. In antisense therapeutics, disease- associated genes or their RNA transcripts are targeted by antisense molecules designed to inhibit their expression. Some of the primary targets for therapeutic antisense design have been proto-oncogenes, such as c-*myc,* N-*myc,* c-*myb,* c-*fos,* N-*ras,* c-H-*ras,* BCL-2, c-*raf*-1, *cdc*-2 and c-*mos* and some of the DNA and RNA viruses. However, antisense therapeutic agents have also been tested or are currently in clinical trials against such diverse conditions as rheumatoid arthritis, restenosis after coronary angioplasty, IgE-induced allergic reactions, renal transplant rejection, Crohn's disease, and psoriasis.

Much of the current antisense research has focused on designing more stable, noncytotoxic ODNs for therapeutic use that are specific for the target nucleic acids. ODNs have been the most frequent form of antisense molecule used because of the greater intracellular stability of antisense DNA as compared to antisense RNA. Most ODNs in use today are relatively short (12-25 nucleotides) and are chemically modified to increase their resistance to intracellular nucleases and to increase their affinity for the target molecules. Unfortunately, these chemical modifications are often accompanied by an increase in cytotoxity, which limits the use of such ODNs *in vivo.* However, one group of ODNs, the phosphorothioate ODNs, has exhibited minimal cytotoxicity and a high level of intracellular stability. In these ODNs, one of the oxygen atoms of the phosphate group normally found in the nucleic acid backbone is replaced with a sulfur atom.

Chemically unmodified antisense has also been utilized against a variety of RNAs with varying degrees of success. One way of producing this kind of antisense is by reverse transcribing a complementary DNA (cDNA) from a target mRNA and then cloning this cDNA into an expression vector in an antisense orientation. The cloned nucleotide sequence is therefore reversed as compared with the normal sequence and RNA transcripts produced from this cloned sequence are able to hybridize with the target mRNA. The expression vector is then either transfected or injected into the target cells. These vectors are transient, but remain within the cell long enough for antisense RNA transcription to occur.

There are several potential problems that must be dealt with in designing a successful antisense molecule. One consideration must be the stability of the antisense RNA in the host cell environment. Normal cellular mRNAs have variable degrees of stability within the cytoplasm, depending upon the presence of a 5' cap, both 5' and 3' UTR regulatory elements, and a poly(A) tail of variable length. Another design consideration must be the secondary and tertiary structures formed by both the target RNA and the antisense RNA. A high degree of complementarity, additionally, is important.

Antisense length also seems to be important. Specificity for a particular target RNA requires an antisense length of at least 11 to 15 nucleotides. Below that length, the antisense tends not to bind. As antisense length increases, the affinity of the antisense for its target also potentially increases because of the increased number of hydrogen bonds possible between the two molecules. There are dangers though in increasing antisense length--the antisense RNA may develop a higher degree of secondary structure which may affect its ability to stably bind to the target. Affinity is determined not only by antisense length but also by the GC versus AU content of the duplex. A high GC content allows the antisense to bind strongly to target RNAs even if there is a partial mismatch between the two molecules, whereas an antisense RNA with high AU content may not bind strongly to its target sequence.

Investigators have developed some optimization rules for antisense design. They include the following: (1) avoid targeting long stems but instead target single stranded areas, short stems, and bulges; (2) avoid antisense designs that contain extremely stable secondary or tertiary structures; (3) choose antisense lengths that allow all bases to bind the target. Again, the ribosome's ability to unwind double stranded structures within its template RNA must be considered.

The present invention concerns novel antisense compositions and for use in methods for treating cancer.

### Summary of the Invention

The present invention provides compositions and methods for inhibiting expression of universal target sequences in a cell, *e.g.,* a tumor cell.

The invention provides an antisense nucleic acid consisting of a sequence complementary to a region in a target mRNA, wherein the region is limited to a poly A tail and excludes AUG, 5' non-translated sequences, translation initiation factor binding sites, ribosome subunit binding sites, Shine Dalgarno sequence, 3' nontranslated sequences, poly-addition site, 3' cleavage site, coding region, intron, intron branch site, intron/exon junction, and splice sequence; or an expression vector that produces the antisense nucleic acid consisting of a sequence complementary to a region in a target mRNA, wherein the region is limited to a poly A tail, and excludes AUG, 5' non-translated sequences, translation initiation factor binding sites, ribosome subunit binding sites, Shine Dalgarno sequence, 3' nontranslated sequences, poly-addition site, 3' cleavage site, coding region, intron, intron branch site, intron/exon junction, and splice sequence for use in a method of treating a tumor, whereby said antisense nucleic acid or said vector is administered into the tumor of a subject.

The antisense nucleic acid or the expression vector is preferably injected into the tumor.

The invention further provides a pharmaceutical composition comprising an antisense nucleic acid consisting of a sequence complementary to a region in a target mRNA, wherein the region is limited to a poly A tail and excludes AUG, 5' non-translated sequences, translation initiation factor binding sites, ribosome subunit binding sites, Shine Dalgarno sequence, 3' nontranslated sequences, poly-addition site, 3' cleavage site, coding region, intron, intron branch site, intron/exon junction, and splice sequence; or
an expression vector that produces the antisense nucleic acid consisting of a sequence complementary to a region in a target mRNA, wherein the region is limited to a poly A tail, and excludes AUG, 5' non-translated sequences, translation initiation factor binding sites, ribosome subunit binding sites, Shine Dalgarno sequence, 3' nontranslated sequences, poly-addition site, 3' cleavage site, coding region, intron, intron branch site, intron/exon junction, and splice sequence for use in a method of treating a tumor, whereby said pharmaceutical composition is administered into the tumor of a subject.

Said pharmaceutical composition is preferably injected into the tumor in a subject.

A prefered subject is a human.

It is further prefered that the antisense nucleic acid or expression vector is formulated in a nanoparticle or liposome.

Further embodiments and the full scope of applicability of the present invention will become apparent from the detail description given hereinafter.

The sequence of the antisense nucleic acid is complementary to a region of the target nucleic acid that is limited to a poly A tail, and excludes a 5' non-translated region, an AUG, a translation initiation factor binding sequence, a ribosome subunit binding sequence, a Shine Dalgarno sequence, a 3' non-translated sequence, a poly-addition site, a 3' mRNA cleavage site, a coding region, or an intron, intron branch, intron/exon junction, and a splice sequence. In still further embodiments of the invention, the antisense nucleic acid may be about 20, 25, 30, 40, 50, 100, 200, 500, 1000, 1500, 2000, or about 4000 bases or longer. The antisense nucleic acid may be comprised in an expression vector.

In some embodiments the target nucleic acid is mRNA. In other embodiments the invention encompasses a method of inhibiting translation of mRNA.

In further embodiments, the target nucleic acid is in a cell. The type of such cell may be, for example, a human cell.

In some embodiments, described is a method for killing a tumor cell comprising contacting a nucleic acid of said cell with an antisense nucleic acid comprising a sequence complementary to a region in a target nucleic acid, wherein the target nucleic acid is a nucleic acid having a poly A tail. The cell may be an animal cell and the target nucleic acid may be mRNA. In one embodiment, the killing comprises inhibiting cell replication. The antisense nucleic acid may be comprised in an expression vector.

In still another embodiment, the invention comprises a pharmaceutical composition comprising an antisense nucleic acid comprising a sequence complementary to a region in a target nucleic acid, wherein the region is limited to a poly A tail and excludes AUG, 5' non-translated sequences, translation initiation factor binding sites, ribosome subunit binding sites, Shine Dalgarno sequence, 3' nontranslated sequences, poly-addition site, 3' cleavage site, coding region, intron, intron branch site, intron/exon junction, and splice sequence. In another embodiment of the invention, the pharmaceutical composition is dispersed in a pharmacologically acceptable buffer, diluent or excipient. In the pharmaceutical composition, the antisense nucleic acid may be comprised in an expression vector, may further be of any type contemplated by the invention.

Further described is a cell comprising an antisense nucleic acid in accordance with the invention. The cell may comprise a eukaryotic cell, including an animal cell, preferably a human cell.

### Brief Description of the Drawings

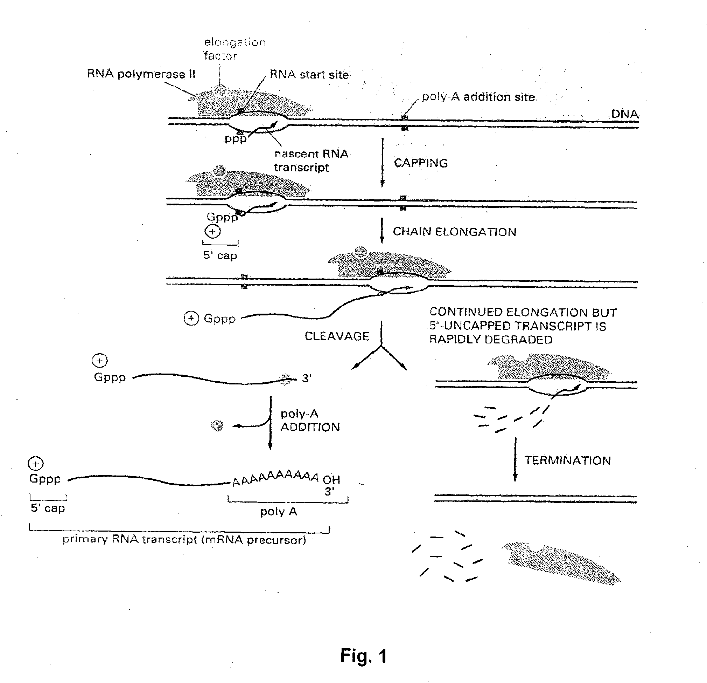

Figure 1. Synthesis of a primary RNA transcript (an mRNA precursor) by RNA polymerase II. This diagram starts with a polymerase that has just begun synthesizing an RNA chain. Recognition of a poly-A addition signal in the growing RNA transcript causes the chain to be cleaved and then polyadenylated as shown.

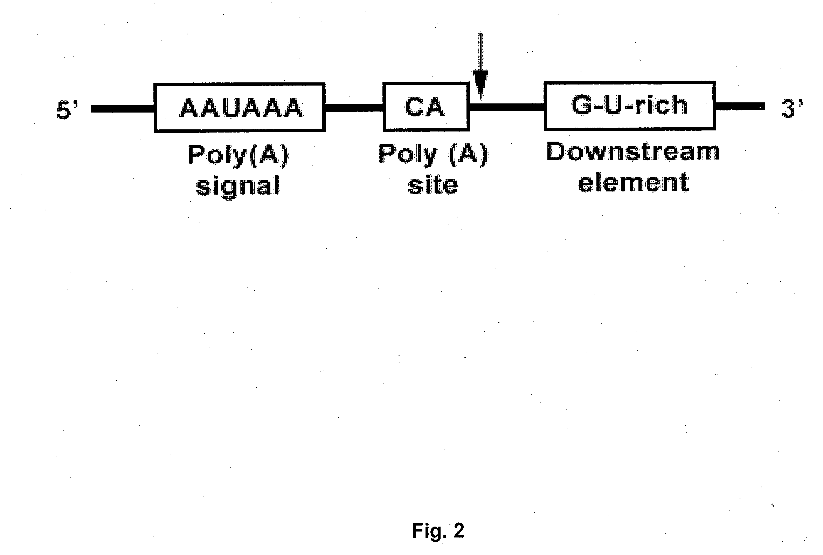

Figure 2. Polyadenylation signal and polyadenylation site. Arrow, site of RNA cleavage.

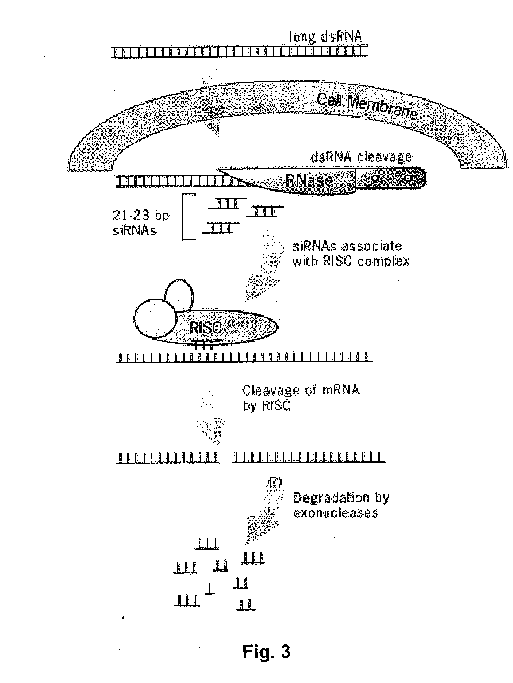

Figure 3. General steps and methods of RNA silencing. In all RNA silencing pathways, double-stranded RNA (dsRNA) is processed to a small RNA which is assembled with RISC into a silencing complex that specifically represses expression or function of a target gene or genomic region by cleaving the corresponding mRNA.

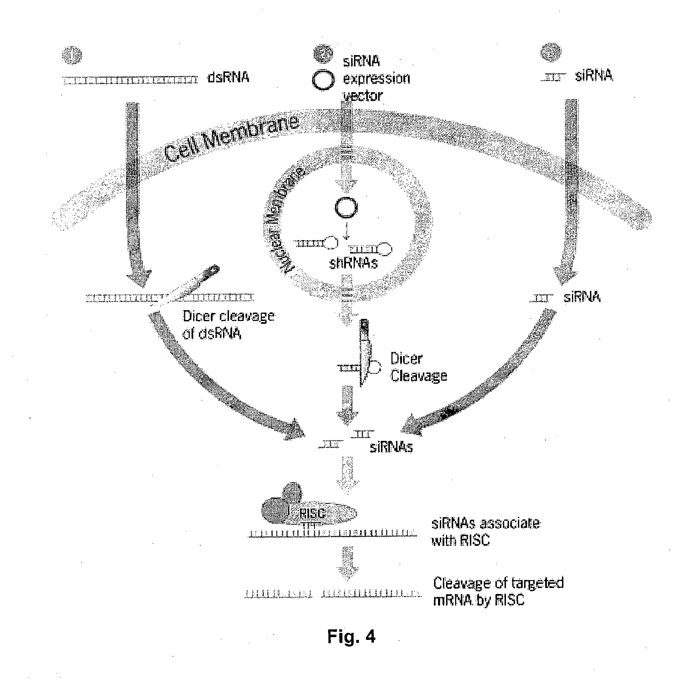

Figure 4. Three ways to trigger the RNAi pathway. In mammalian cells, RNAi is typically induced by the use of siRNAs. There are two general methods for producing siRNAs in cells: delivery of synthetic siRNAs, and introduction of a DNA construct that expresses short hairpin RNA sequences (shRNA) that are processed to siRNAs within the cell.

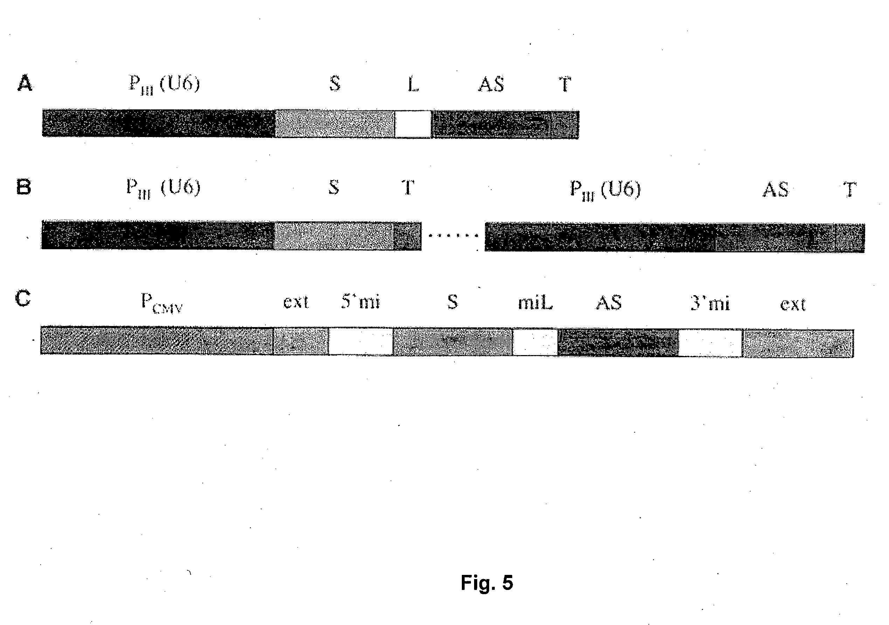

Figure 5. Schematic representation of expression cassettes for shRNA (A), siRNA (B) and miRNA (C). P_{III} (U6): pol III promoter (U6), P_{CMV}: pol II promoter (CMV), S: siRNA sense strand, AS: siRNA antisense strand, L: loop, T: terminator, 5'mi: 5' pri-miRNA sequence, 3'mi: 3' pri-miRNA, ext: extraneous transcript sequences. The 5'mi and 3'mi sequences direct proper excision of the siRNA from the heterologous transcript.

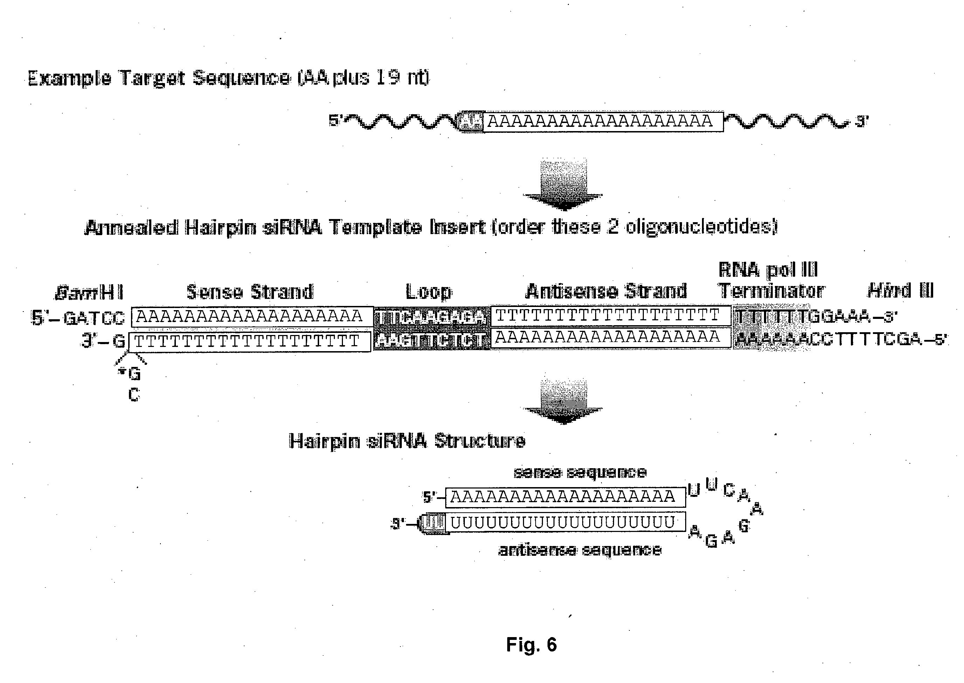

Figure 6. Example siRNA template design (Pol III promoter). Example target sequence; siRNA template insert (top strand), (bottom strand); hairpin RNA.

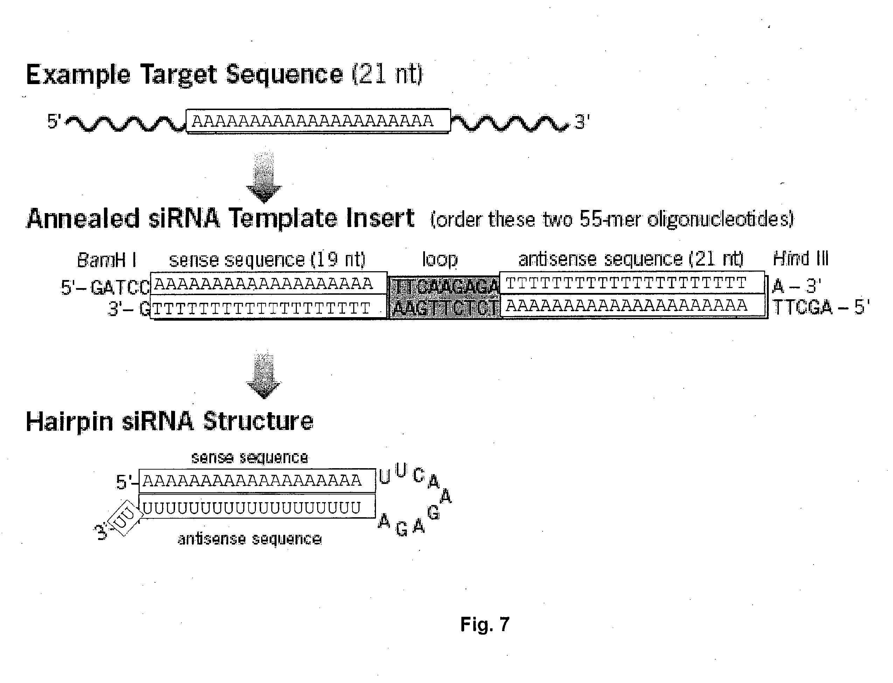

Figure 7. Example siRNA template design (CMV promoter). Example target sequence, (SEQ ID NO: __); siRNA template insert (top strand), (SEQ ID NO: __), (bottom strand), (SEQ ID NO: __); hairpin RNA, (SEQ ID NO: __).

### Detailed Description of the Preferred Embodiment

As disclosed, this technology can act like a surgical tool to debulk tumors.

By expressing a poly dT to be followed by a poly A, you can use the Dicer Method, i.e., similar to siRNA, to block expression of almost all genes if the poly T is being expressed by a very strong promoter; for this purpose we have several promoter candidates, all expressing housekeeping genes, i.e., ribosomal RNA promoters, collagenase promoter, ubiquitin promoter, albumin promoter, and the like. When we express the poly dT, it will hybridize to the poly A (the resulting expressed poly U will hybridize to the poly A of all the mRNAs that have poly A tails), therefore shutting down protein expression in the cells and causing their death by preventing their normal metabolic function.

By expressing an oligo U in the cell and placing the expression unit in a replicating adenovirus (poxvirus, retrovirus, or the like), we could inject the tumors, even those that are not accessible to surgery, and by adenovirus replicating cover the whole tumor. What can be done is that once you have adequate spread of the virus in the tumor, you kill the virus complimentary partner by placing a suicide gene in it. For an additional safety factor, we can utilize two suicide genes, placing one in each of the two defective viruses.

### Molecular Nano-Surgery Technology

Many groups use oncolytic (tumor busting) virus vectors containing a specific gene or just a conditional virus to kill tumor cells. These molecules can be injected into the tumor. Alternatively, if the vectors (virus) can be activated only inside the tumor the molecules can be injected intravenously. One problem with this approach is that if the tumor develops a by pass for these molecules the therapy becomes ineffective.

Eukaryotic cells, in order to function, must produce different gene products. They achieve this function by sending messages from the genes to cytoplasm where different proteins and enzymes are made using the message blueprints. The genes send their messages to the cytoplasm in the form of messenger RNA (mRNA). Almost all eukaryotic mRNAs have a unique characteristic. They have a tail of poly A, which is a signal that is essential for the proper function of the cell machinery to utilize the mRNA blueprints.

In this new approach we are taking the approach of using the poly A tail to destroy the tumor. In our approach we are proposing to use different molecules to disrupt the function of the poly A and use it to destroy the tumor. Some but not all of these approaches are listed below:

Place oligo-T or oligo-U or their derivatives to hybridize to the poly A tails of the mRNA. These molecules can be placed in liposome and seeded inside the tumor when damage to the adjacent organs or tissues makes it impossible to remove the tumor or its remnants.

Using the same approach, express the oligo-U inside the tumor by using a vector.

Because the poly A tail is a universal characteristic of the eukaryotic mRNA it is almost impossible for the tumor to develop a rescue mechanism against this approach. However, it should also be mentioned here that this approach is not immunogenic. It merely shrinks the tumor to a more manageable size. For example, when because of the spread of the tumor and its proximity to a vital organ it is impossible to perform surgery utilization of this approach can shrink the tumor in such a way that would allow removal of the remaining tumor.

An additional advantage of this approach is that it can be used in conjunction with chemotherapy, thus if the cells have developed a multiple drug resistance, utilization of this approach with chemotherapy will sensitize the cells to chemotherapy again.

### The Precursors of Messenger RNA are Covalently Modified at Both Ends

In eucaryotes, mature mRNA is produce in several steps. The RNA molecules freshly synthesized by RNA polymerase II in the nucleus are known as primary transcripts; the collection of such transcripts was originally called heterogeneous nuclear RNA (hnRNA) because of the large variation in RNA size, contrasting with the more uniform and smaller size of the RNA sequences actually needed to encode proteins. As they are being synthesized, these transcripts are covalently modified at both their 5' end and their 3' end in ways that clearly distinguish them from transcripts made by other RNA polymerases (**Fig. 1**).

The 5' end of the RNA molecule (which is the end synthesized first during transcription) is first capped by the addition of a methylated G nucleotide. Capping occurs almost immediately, after about 30 nucleotides of RNA have been synthesized, and it involves condensation of the triphosphate group of a molecule of GTP with a diphosphate left at the 5' end of the initial transcript. This 5' cap will later play an important part in the initiation of protein synthesis; it also seems to protect the growing RNA transcript from degradation.

The 3' end of most polymerase II transcripts is defined not by the termination of transcription but by a second modification in which the growing transcript is cleaved at a specific site and a poly-A tail is added by a separate polymerase to the cut 3' end. The signal for the cleavage is the appearance in the RNA chain of the polyadenylation signal AAUAAA located 10 to 30 nucleotides upstream from the site of cleavage, plus a less well-defined downstream sequence. Immediately after cleavage, a poly A polymerase enzyme adds 100 to 200 residues of adenylic acid (as poly A) to the 3' end of the RNA chain to complete the primary RNA transcript. Meanwhile, the polymerase fruitlessly continues transcribing for hundreds or thousands of nucleotides until termination occurs at one of several later sites; the extra piece of functionless RNA transcript thus generated presumably lacks a 5' cap and is rapidly degraded (**Fig. 1**).

Referring to **Fig. 2**, the polyadenylation signal AAUAAA is located downstream of the 3' exon. The polyadenylation site is the site at which polyadenine is added to mRNA. It is localized downstream of the polyadenylation signal. The sequence immediately 5' to the site of RNA cleavage is frequently (but not always) CA. A G-U-rich element usually lies just downstream of the site of cleavage end important for efficient processing (Manley, J.L. and Takagaki, Y. 1996 Science 274:1481-1482).

### Blocking of Gene Expression in Eukaryotic Cells

The embodiments of the present invention contemplate that targeting the poly(A) site abrogates gene expression as effectively as targeting a sensitive internal coding sequence.

### Definitions

Unless defined otherwise, technical and scientific terms used herein have the same meaning as commonly understood by one of ordinary skill in the art to which this invention belongs. See, e.g., Singleton P and Sainsbury D., Dictionary of Microbiology and Molecular Biology 3rd ed., J. Wiley & Sons, Chichester, New York, 2001.

The transitional term "comprising" is synonymous with "including," "containing," or "characterized by," is inclusive or open-ended and does not exclude additional, unrecited elements or method steps.

The transitional phrase "consisting of" excludes any element, step, or ingredient not specified in the claim, but does not exclude additional components or steps that are unrelated to the invention such as impurities ordinarily associated therewith.

The transitional phrase "consisting essentially of" limits the scope of a claim to the specified materials or steps and those that do not materially affect the basic and novel characteristic(s) of the claimed invention.

As used herein, the term "vector" refers to the plasmid, virus or phage chromosome used in cloning to carry the cloned DNA segment. Vectors capable of directing the expression of genes to which they are operatively linked are referred to herein as "expression vectors". Another type of vector is a genomic integrated vector, or "integrated vector", which can become integrated into the chromosomal DNA of the host cell. Another type of vector is an episomal vector, i.e., a nucleic acid capable of extra-chromosomal expression. In the present specification, "plasmid" and "vector" are used interchangeably unless otherwise clear from the context.

As used herein, the term "nucleic acid" refers to polynucleotides such as deoxyribonucleic acid (DNA), and, where appropriate, ribonucleic acid (RNA). The term should also be understood to include, as applicable to the embodiment being described, single-stranded (such as sense or antisense) and double-stranded polynucleotides.

As used herein, the term "gene" or "recombinant gene" refers to a nucleic acid comprising an open reading frame encoding a polypeptide of the present invention, including both exon and (optionally) intron sequences. A "recombinant gene" refers to nucleic acids encoding such polypeptides that may optionally include intron sequences that are derived from chromosomal DNA. The term "intron" refers to a DNA sequence present in a given gene that is not present in the mature RNA and is generally found between exons.

As used herein, "cell" refers to a eukaryotic cell. Typically, the cell is of animal origin and can be somatic cells. Suitable cells can be of, for example, mammalian, avian or plant origin. Examples of mammalian cells include human, bovine, ovine, porcine, murine, and rabbit cells. Where the cell is a somatic cell, the cell can be, for example, an epithelial cell, fibroblast, smooth muscle cell, blood cell (including a hematopoietic cell, red blood cell, T-cell, B- cell, etc.), tumor cell, cardiac muscle cell, macrophage, dendritic cell, neuronal cell (e.g., a glial cell or astrocyte), or pathogen-infected cell (e.g., those infected by bacteria, viruses, virusoids, parasites, or priors).

The term "RNA interference" or "RNAi" refers to the silencing or decreasing of gene expression by siRNAs. It is the process of sequence- specific, post-transcriptional gene silencing in animals and plants, initiated by siRNA that is homologous in its duplex region to the sequence of the silenced gene.

As used herein, the terms "RNA" and "RNA molecule(s)" are used interchangeably to refer to RNA that mediates RNA interference. These terms include double-stranded RNA, single-stranded RNA, isolated RNA (partially purified RNA, essentially pure RNA, synthetic RNA, recombinantly produced RNA etc.), as well as altered RNA that differs from naturally occurring RNA by the addition, deletion, substitution and/or alteration of one or more nucleotides.

The term "loss-of function", as it refers to genes inhibited by the RNAi method of the present invention, refers to diminishment in the level of expression of a gene when compared to the level in the absence of the dsRNA constructs.

The term "expression" with respect to a gene sequence refers to transcription of the gene and, as appropriate, translation of the resulting mRNA transcript to a protein. Thus, as will be clear from the context, expression of a protein coding sequence results from transcription and translation of the coding sequence.

By "inhibit" it is meant that the activity of a gene expression product or level of RNAs or equivalent RNAs encoding one or more gene products is reduced below that observed in the absence of the nucleic acid molecule of the invention.

The term "silencing" as used herein refers to suppression of expression of the (target) gene. It does not necessarily imply reduction of transcription, because gene silencing is believed to operate in at least some cases post-transcriptionally. The degree of gene silencing can be complete so as to abolish production of the encoded gene product (yielding a null phenotype), but more generally the gene expression is partially silenced, with some degree of expression remaining (yielding an intermediate phenotype). The term should not therefore be taken to require complete "silencing" of expression.

As used herein, "introducing" refers to the transfer of a nucleic acid molecule from outside a host cell to inside a host cell. Nucleic acid molecules can be "introduced" into a host cell by any means known to those of skill in the art, for example as taught by Sambrook et al., Molecular Cloning: A Laboratory Manual, Vol. 1-3, Cold Spring Harbor Laboratory Press, Cold Spring Harbor, N.Y., 2001. Means of "introducing" nucleic acids into a host cell include, but are not limited to heat shock, calcium phosphate transfection, electroporation, lipofection, and viral-mediated gene transfer.

As used herein, the term "transfection" refers to the introduction of a nucleic acid, e.g., an expression vector, into a recipient cell by nucleic acid-mediated gene transfer. "Transformation" as used herein, refers to a process in which a cell's genotype is changed as a result of the cellular uptake of exogenous DNA or RNA, and, for example, the transformed cell expresses a dsRNA construct.

As used herein, the term "infection" means the introduction of a nucleic acid by a virus into a recipient cell or organism. Viral infection of a host cell is a technique which is well established in the art and can be found in a number of laboratory texts and manuals such as Sambrook et al., Molecular Cloning: A Laboratory Manual, Vol. 1-3, Cold Spring Harbor Laboratory Press, Cold Spring Harbor, N.Y., 2001.

### Effective gene silencing

Described are methods for attenuating or inhibiting gene expression in a cell using gene- targeted double stranded RNA (dsRNA). The dsRNA contains a nucleotide sequence that hybridizes under physiologic conditions of the cell to the nucleotide sequence of at least a portion of the target mRNA of the gene to be inhibited (target gene).

The siRNAs will typically comprise 15-40 nucleotides comprising at least two parts, a first part comprising a sequence corresponding to at least a part of a poly A sequence and optionally a second part comprising unique sequence corresponding to 9-34 contiguous or non-contiguous nucleotides from the region adjacent to said poly A sequence. The unique sequences adjacent to the poly A sequence can be on the 3' side, on the 5' side or both.

Further described are poly A sequences that comprise a shorter or longer number of nucleotides.

Poly A sequence serves as a universal target sequence and optionally non coding sequences operate as unique gene specific sequences for siRNA activity.

According to current knowledge, in 68% of the human genes, the 6 nucleotides AAUAAA of the consensus sequence of the Poly(A) signal are flanked by unique sequences of at least 15 nucleotides. Most of the remaining genes (32%) include multicopy genes or mRNA splice variants of the same gene.

The method described herein does not require 100% sequence identity between the siRNA and the target gene. By utilizing bioinformatics tools, the sequence can contain mismatch pairs of nucleotides. Thus, the methods have the advantage of being able to tolerate some sequence variations that might be expected due to genetic mutation, strain polymorphism, or evolutionary divergence.

The target gene can be a gene derived from the cell (*i.e.,* a cellular gene), an endogenous gene (*i.e.,* a cellular gene present in the genome), a transgene (*i.e*., a gene construct inserted at an ectopic site in the genome of the cell), or a gene from a pathogen (such as a virus, bacterium, fungus or protozoan) which is capable of infecting an organism. Depending on the particular target gene and the dose of double stranded RNA material delivered, this process can provide partial or complete loss of function for the target gene.

Inhibition of gene expression refers to the absence (or observable decrease) in the level of protein and/or mRNA product from a target gene. Specificity refers to the ability to inhibit the target gene without manifesting effects on other genes of the cell. According to the present invention, quantification of the amount of gene expression allows one to determine the degree of inhibition which is greater than 50%, preferably 65%, more preferably 75%, and most preferably 95% and more.

### Designing siRNAs

Computational analysis demonstrated a high conservation of the poly(A) signal of both cell and viral mRNAs. Investigators found that 97.45% of human mRNA 3' untranslated regions (UTRs) harbor an AAUAAA sequence, which is flanked by unique sequences of at least 15 bases. The remaining 3' UTRs, that have redundant poly(A) regions, include poly(A) regions that are shared among several genome locations, but are annotated to be producing the same protein. Many of the others belong to different genes that produce different proteins, but belong to the same protein family.

Exemplary siRNAs based on the human mRNA 3' UTR sequences of a broad range of gene functions designed according to the principles of the present invention are presented in Table 1. The 3' UTR sequence is located within the 10 to 30 nucleotides between the AATAAA polyadenylation signal and the site of cleavage at which the growing transcript is cleaved and at which the poly A tail is added to complete the primary RNA transcript. The inhibition of the exemplary gene by the siRNA will typically reduce the phenotypic expression of the gene of interest in eukaryotic cells. However, besides the expected loss of function phenotype, previously unknown functions or phenotypes may become apparent upon gene silencing. It will be appreciated by the skilled artisan that siRNA may be used to decipher gene pathways and interactions or to confirm interactions.

**Table 1. Exemplary siRNAs**

| **Gene Symbol** | **Gene Product** | **Reference Sequence Database No.** | **Gene Function** | **siRNA sequences** | **SEQ ID NO.** |
|---|---|---|---|---|---|
| BCL2L2 | BCL2-like2 | NM_004050 | Anti-apoptosis | Sense; | |
| | | | | 5'-taaagcccagaagtttaatgag-3' | 1 |
| | | | | Antisense; | |
| | | | | 5' ctcattaaacttctgggcttta-3' | 2 |
| CXCR3 | Chemokine (C-X-C motif) receptor 3 | NM_001504 | Antimicrobial humoral response | Sense; | |
| | | | | 5'-caagatcgtcaggacc-3' | 3 |
| | | | | Antisense; | |
| | | | | 5'-ggtcctgacgatcttg-3' | 4 |
| DIPA | Hepatitis delta antigen-interacting protein A | NM_006848 | Regulates early events of adipogenesis | Sense; | |
| | | | | 5'-cccggacggaagcgga-3' | 5 |
| | | | | Antisense; | |
| | | | | 5'-tccgcttccgtccggg-3' | 6 |
| GDF3 | Growth differentiation factor 3 | NM_020634 | Cell growth and maintenance | Sense; | |
| | | | | 5'-actacctatctggtttatgaccacttagatcgaaat gtca-3' | 7 |
| | | | | Antisense; | |
| | | | | 5'-tgacatttcgatctaagtggtcataaaccagatag gtaga-3' | 8 |
| GSTA3 | Glutathione S-transferase A3 | NM_000847 | Response to stress | Sense; | |
| | | | | 5'-aactcctatttgctaactta-3' | 9 |
| | | | | Antisense; | |
| | | | | 5'-taagttagcaaataggagtt-3' | 10 |
| HEBP1 | Heme binding protein 1 | NM_015987 | Circadian rhythm | Sense; | |
| | | | | 5'-aggcattgacttaaacagctgagacaaa-3' Antisense; | 11 |
| | | | | 5'-tttgtctcagctgtttaagtcaatgcct-3' | 12 |
| INSM1 | Insulinoma associated 1 | NM_002196 | Regulation of transcription | Sense; | |
| | | | | 5'-atattttcaaagtcaaa-3' | 13 |
| | | | | Antisense; | |
| | | | | 5'-tttgactttgaaaatat-3' | 14 |
| MYC | V-myc myelocytomatosis viral oncogene homolog (avian) | NM_002467 | Cell proliferation | Sense; | |
| | | | | 5'-ataactggcaaatatatcattgagccaaa-3' | 15 |
| | | | | Antisense; | |
| | | | | 5'-tttggctcaatgatatatttgccagttat-3' | 16 |
| PSMDI | Proteasome (prosome, macropain) 26S subunit, non ATPase,1 | NM_002807 | Regulation of | Sense; | |
| | | | cell cycle | 5'-tataagatctccagatggacaag-3' | 17 |
| | | | | Antisense; | |
| | | | | 5'-cttgtccatctggagatcttata-3' | 18 |
| TNFRS F10D | Tumor necrosis factor receptor superfamily, member 10d, decoy with truncated death domain | NM_003840 | Apoptosis | Sense; | |
| | | | | 5'-tatgaaacctcatattaaaa-3' | 19 |
| | | | | Antisense; | |
| | | | | 5'-ttttaatatgaggtttcata-3' | 20 |
| WAS | Wiscott-Aldrich syndrome protein eczema-thrombocyto penia | NM_000377 | Actin polymerizatio n and/or depolymerization | Sense; | |
| | | | | 5'-agaattgtctttctgtctctctat-3' | 21 |
| | | | | Antisense; | |
| | | | | 5'-atagagagacagaaagacaattct-3' | 22 |

### Applications of siRNA

Further described is a variety of applications in which it is desired to modulate, e.g., one or more target genes, viral replication of a pathogenic virus, etc., in a whole eukaryotic organism, e.g., a mammal or a plant, or portion thereof, e.g., tissue, organ, cell, etc. In such methods, an effective amount of an RNAi active agent is administered to the host or introduced into the target cell. The term "effective amount" refers to a dosage sufficient to modulate expression of the target viral gene(s), as desired, e.g., to achieve the desired inhibition of viral replication. As indicated above, the subject methods are employed to reduce expression of one or more / target genes in the host in order to achieve a desired therapeutic outcome.

When the target gene is a viral gene, e.g., when inhibition of viral replication is desired, the target viral gene can be from a number of different viruses. Representative viruses include, but are not limited to: HBV, HCV, HIV, influenza A, Hepatitis A, picornaviruses, alpha-viruses, herpes viruses, and the like.

The methods described herein are also suitable for inhibiting the expression of a target gene in a tumor cell. The present invention relates to any type of cancer including breast cancer, cancers of the head and neck including various lymphomas such as mantle cell lymphoma, non-Hodgkins lymphoma, adenoma, squamous cell carcinoma, laryngeal carcinoma, cancers of the retina, cancers of the esophagus, multiple myeloma, ovarian cancer, uterine cancer, melanoma, colorectal cancer, bladder cancer, prostate cancer, glioblastoma, lung cancer (including non-small cell lung carcinoma), pancreatic cancer, cervical cancer, head and neck cancer, skin cancers, nasopharyngeal carcinoma, liposarcoma, epithelial carcinoma, renal cell carcinoma, gallbladder adeno carcinoma, parotid adenocarcinoma, endometrial sarcoma, and multidrug resistant cancers.

### Approaches for synthetic siRNA and vector-mediated RNAi

### 1. Introduction

Referring to **Fig. 3**, RNA interference (RNAi) is a process, first described in the worm *Caenorhabditis elegans,* whereby the presence or introduction of long double-stranded RNA (dsRNA) in cells results in the degradation of homologous mRNA (A. Fire et al. Nature 391 (1998), pp. 806-811) and (G.J. Hannon Nature 418 (2002), pp. 244-251). Long dsRNA is processed to 21-23 bp short interfering RNA (siRNA) with 2 nt 3' overhangs by the RNAse III-like protein Dicer (E. Bernstein et al. Nature 409 (2001), pp. 363-366). These cleavage products are subsequently incorporated into the RNA-induced silencing complex (RISC) (S.M. Hammond et al. Nature 404 (2000), pp. 293-296). Delivery of chemically synthesized short interfering RNAs, mimicking Dicer cleavage substrates, results in sequence-specific, robust silencing of the expression of the corresponding endogenous gene (S.M. Elbashir et al. Nature 411 (2001), pp. 494-498), thus bypassing the non-specific inhibitory mechanisms elicited by longer dsRNA in mammalian cells (B.R. Williams Oncogene 18 (1999), pp. 6112-6120). RNAi can also be induced by endogenous expression of short hairpin RNAs (shRNAs) (T.R. Brummelkamp et al. Science 296 (2002), pp. 550-553). shRNAs are structurally related to a highly conserved class of small RNAs known as microRNAs (miRNAs) that mediate RNAi through a translational inhibition mechanism involving imperfect complementarity to sites in the 3' UTR of target genes (L. He and G.J. Hannon, Nat Rev Genet 5 (2004), pp. 522-531). miRNAs are transcribed as precursors that are first processed in the nucleus by the RNase III protein Drosha in the Microprocessor complex (Y. Lee et al. Nature 425 (2003), pp. 415-419), (A.M. Denli et al. Nature 432 (2004), pp. 231-235) and (R.I. Gregory et al. Nature 432 (2004), pp. 235-240). The product of Drosha-mediated processing, pre-miRNA, is exported to the cytoplasm by Exportin 5 (E. Lund et al. Science 303 (2004), pp. 95-98), for further processing by Dicer to the mature miRNA (Y. Lee et al. EMBO J. 21 (2002), pp. 4663-4670). One of the strands is incorporated into a RISC-like silencing complex (Z. Mourelatos et al. Genes Dev. 16 (2002), pp. 720-728).

Referring to **Fig. 4**, we describe the various methodologies for eliciting RNAi by either synthetic or expressed RNAi effector molecules.

### 2. Synthetic siRNA-mediated RNAi

2.1. Enzymatically generated siRNA. The most cost effective and quickest method for siRNA synthesis is T7 phage RNA polymerase mediated *in vitro* transcription from short double-stranded oligo cassettes containing the promoter sequence immediately upstream of the siRNA strand template sequence to be transcribed (M. Sohail et al. Nucleic Acids Res. 31 (2003), p. e38) and (L. Scherer and J.J. Rossi Biotechniques 36 (2004), pp. 557-561). The siRNA strands are synthesized in separate reactions and hybridized before purification. Once the template oligos are available, template preparation (annealing), *in vitro* transcription, siRNA annealing, and purification can be completed within 24 h. The siRNAs synthesized by this method frequently contain a GGG leader sequence (deriving from the promoter) as well as a 5' triphosphate group (D.H. Kim et al. Nat. Biotechnol. 22 (2004), pp. 321-325). The hybridized siRNA thus needs to be processed by T1 ribonuclease to remove the single stranded 5' GGG overhang. Replacement of the 3' UU residues with 3' AA that cannot form wobble base pairs with the 5'GG improves processing and reduces the potential for interferon induction (D.H. Kim et al. Nat. Biotechnol. 22 (2004), pp. 321-325). Changing the siRNA from 19 + UU to 21 + AA, with a 21 nt target complementary duplex region is also associated with enhanced RNAi activity.

Different siRNA sequences display widely differing efficacies, requiring screening of multiple sequences (L. Scherer and J.J. Rossi, Biotechniques 36 (2004), pp. 557-561), (T. Holen et al. Nucleic Acids Res. 30 (2002), pp. 1757-1766) and (L.J. Scherer and J.J. Rossi, Nat. Biotechnol. 21 (2003), pp. 1457-1465). One way to get around this problem is by application of a pool of enzymatically generated siRNAs. Dicer, an RNase III family enzyme, cleaves *in vitro* transcribed long dsRNA into a pool of siRNAs suitable for gene silencing (H. Zhang et al. EMBO J. 21 (2002), pp. 5875-5). Therefore, several groups have produced a recombinant version of Dicer and used it to *digest in vitro* transcribed dsRNAs into a complex pool of siRNAs (d-siRNA) (J.W. Myers and J.E. Ferrell, Methods Mol. Biol. 309 (2005), pp. 93-196). Nearly every pool of d-siRNAs is capable of eliciting specific gene silencing. This approach eliminates the need to identify an individual effective siRNA and has proven to be useful for transiently silencing many endogenous genes in several types of cells.

2.2. Chemically synthesized siRNAs. Chemically synthesized siRNAs represent the gold standard for RNAi applications. They are of a uniform composition and can be synthesized at higher amounts and with a wider range of chemical modifications than by other methods (M. Amarzguioui et al. Nucleic Acids Res. 31 (2003), pp. 589-595), (D.A. Braasch et al. RNA, Biochemistry 42 (2003), pp. 7967-7975) and (Y.L. Chiu and T.M. Rana et al. RNA 9 (2003), pp. 1034-1048). The disadvantages include higher cost and increased synthesis time. Initial studies in Drosophila melanogaster embryo lysates concluded that 21 nt siRNAs with 2 nt 3' overhangs were the most efficient triggers of sequence-specific mRNA degradation (S.M. Elbashir et al. EMBO J. 20 (2001), pp. 6877-6888), and most subsequent studies have therefore employed this format. During investigation of cellular interferon induction caused by *in vitro* transcribed siRNAs, investigators observed that some siRNAs of length 25-27 appeared to have greater potency than synthetic 21mer siRNAs directed to the same target site (D.H. Kim et al. Nat. Biotechnol. 22 (2004), pp. 321-325). Synthetic RNA duplexes of varying length containing 3'-overhangs, 5'-overhangs, or blunt ends, were tested for their relative potency in several reporter systems (D.H. Kim et al. Nat. Biotechnol. 23 (2005), pp. 222-226). Using duplex RNA at several concentrations, investigators observed that potency increased with length up to a duplex length of 27 bp. Increased potency was observed even for siRNAs with 5' overhangs or blunt ends (D.H. Kim et al. Nat. Biotechnol. 23 (2005), pp. 222-226). Reduced efficacy was observed for siRNA with longer than 27 bp stems, which also exhibited *slower in vitro* Dicing kinetics. Hannon and colleagues (D. Siolas et al. Nat. Biotechnol. 23 (2005), pp. 227-231) also found synthetic shRNAs with 29-base-pair stems and 2-nucleotide 3' overhangs to be more potent inducers of RNAi than shorter hairpins. Maximal inhibition of target genes was achieved at lower concentrations and silencing persisted longer. Providing the RNAi machinery with a Dicer substrate therefore presumably results in more efficient incorporation of the active 21mer into RISC.

### 3. Vector-based RNAi

The solution to the problem of downregulation of gene expression mediated by siRNA is stable expression of RNAi effector molecules from plasmids or viral vectors. The use of viral vectors, such as lentiviruses and adenovirus (M.J. Li et al., Mol. Ther. 8 (2003), pp. 196-206), (D.A. Rubinson et al., Nat. Genet. 33 (2003), pp. 401-406), (G. Tiscornia et al. Proc. Natl. Acad. Sci. USA 100 (2003), pp. 1844-1848), (C. Shen et al. FEBS Lett. 539 (2003), pp. 111-114) and (M. Li and J.J. Rossi, Methods Mol. Biol. 309 (2005), pp. 261-272), allows easy generation of transgenics of even hard-to-transfect cells. Vector-based RNAi also permits co-expression of reporter genes such as GFP or luciferase, which facilitates tracking and/or selection/enrichment of transfected/transduced cells. Three different strategies exist for vector-based RNAi, involving the expression of molecules that can be classified as shRNA, siRNA and miRNA (**Table 2**). The most commonly used approach involves RNA polymerase III-mediated transcription of short hairpin structures with a stem of 19-29 bp and a short loop of 4-10 nt (T.R. Brummelkamp et al. Science 296 (2002), pp. 550-553), (J. Harborth et al. Antisense Nucleic Acid Drug Dev. 13 (2003), pp. 83-105) and (P.J. Paddison et al. Genes Dev. 16 (2002), pp. 948-958) (**Fig. 5A**). Less commonly, the two strands of an siRNA are transcribed from separate expression units, from either the same or two separate plasmids (J.Y. Yu et al. Proc. Natl. Acad. Sci. USA 99 (2002), pp. 6047-6052) and (N.S. Lee et al. Nat. Biotechnol. 20 (2002), pp. 500-505) (**Fig. 5B**). Finally, the effector molecules may be expressed as a chimera of siRNA and miRNA (Y. Zeng et al. Mol. Cell 9 (2002), pp. 1327-1333) (**Fig. 5C**).

**Table 2. Expression Systems for Vector-based RNAi**

| **Expression unit** | **Promoter** | **Inducible system** | **Refs** |
|---|---|---|---|
| Chimeric miRNA | CMV | Not inducible | Y. Zeng et al. Mol. Cell 9 (2002), pp. 1327-1333; Y. Zeng et al. Proc. Natl. Acad. Sci. USA 100 (2003), pp. 9779-9784; and Y. Zeng and B.R. Cullen, J. Biol. Chem. (2005). |
| tRNA-shRNA fusion | tRNA-Val | Not inducible | K. Oshima et al. Cancer Res. 63 (2003), pp. 6809-6814 |
| shRNA | U6 | Not inducible | P.J. Paddison et al. Genes Dev. 16 (2002), pp. 948-958; and G. Sui et al. Proc. Natl. Acad. Sci. USA 99 (2002), pp. 5515-5520. |
| | | Tetracycline | F. Czauderna et al., Nucleic Acids Res. 31 (2003), p. e127; S. Matsukura et al. Nucleic Acids Res. 31 (2003), p. e77; and X. Lin et al. FEBSLett. 577 (2004), pp. 376-380. |
| | | Ecdysone | S. Gupta et al. Proc. Natl. Acad. Sci. USA 101 (2004), pp. 1927-1932. |
| | | Cre-loxP | V. Kasim, M. Miyagishi and K. Taira, Control of siRNA expression using the Cre-loxP recombination system, Nucleic Acids Res. 32 (2004), p. e66; G. Tiscornia, V. Tergaonkar et al. Proc. Natl. Acad. Sci. USA 101 (2004), pp. 7347-7351; X. Coumoul et al. Nucleic Acids Res. 32 (2004), p. e85; A. Ventura et al. Proc. Natl. Acad. Sci. USA 101 (2004), pp. 10380-10385; H.S. Chang et al. Am. J. Pathol. 165 (2004), pp. 1535-1541 ; and X. Coumoul et al. Nucleic Acids Res. 33 (2005), p. e102. |
| | H1 | Not inducible | T.R. Brummelkamp et al. Science 296 (2002), pp. 550-553. |
| | | Tetracycline | M. van de Wetering et al., EMBO Rep. 4 (2003), pp. 609-615. |
| | | Lac | M. Higuchi et al. Cancer Sci. 95 (2004), pp. 442-447. |
| | 7SK | Not inducible | F. Czauderna et al., Nucleic Acids Res. 31 (2003), p. e127. |
| | | Tetracycline | F. Czauderna et al., Nucleic Acids Res. 31 (2003), p. e127. |
| siRNA | U6 | Not inducible | J.Y. Yu, S.L. DeRuiter and D.L. Turner, Proc. Natl. Acad. Sci. USA 99 (2002), pp. 6047-6052; and N.S. Lee et al. Nat. Biotechnol. 20 (2002), pp. 500-505. |

3.1. Expression strategies for vector-based RNAi. Expression of an shRNA as part of a longer polymerase II derived transcript faces the problem that extraneous sequences might render the heterogeneous transcripts unrecognizable by the cellular RNAi machinery (P.J. Paddison et al. Genes Dev. 16 (2002), pp. 948-958). This is not surprising since near-perfect stem-loop structures are not uncommon in naturally occurring mRNAs, yet do not appear to be processed to siRNA-like molecules. The expression of RNAi effectors as part of more complex transcripts therefore needs to address the problem of proper processing, which may be achieved through incorporation in the primary transcript of naturally occurring signal sequences to direct their processing. Such a strategy is used for expression of siRNA as a part of a polymerase II miRNA transcript. miRNAs are structurally very similar to siRNAs and are incorporated into a RISC-like complex that shares many of the same components as RISC (Z. Mourelatos et al., Genes Dev. 16 (2002), pp. 720-728). Silencing by miRNA occurs at the translational level through imperfect mismatches with the target (J.G. Doench et al .Genes Dev. 17 (2003), pp. 438-442) and (D.P. Bartel and C.Z. Chen, Nat. Rev. Genet. 5 (2004), pp. 396-400). When the target is perfectly complementary, however, miRNAs can mediate cleavage (S. Yekta et al. Science 304 (2004), pp. 594-596). Mature miRNAs can be generated from RNA polymerase II transcribed mRNAs containing irrelevant sequences in addition to the predicted pre-miRNA precursor sequence (Y. Zeng et al. Mol. Cell 9 (2002), pp. 1327-1333) and (Y. Zeng et al. Proc. Natl. Acad. Sci. USA 100 (2003), pp. 9779-9784). While production of mature miRNA requires maintaining the proper precursor stem-loop structure, the exact sequence of this precursor does not appear to be important, and can therefore be replaced with a heterologous stem (**Fig. 5C****)**, enabling the generation of a miRNA-based expression cassette with generalizable targeting properties. Recent work suggests that single stranded extensions to the pre-miRNA hairpin structure are required for full Drosha functionality (Y. Zeng and B.R. Cullen et al. J. Biol. Chem. (2005)). Therefore, to ensure that heterologous miRNAs are properly processed, miRNA-based expression cassettes might, in addition to the pre-miRNA structure, contain 5' and 3' extensions derived from the wild-type miRNA gene to mimic the structure of the wildtype transcript as closely as possible.

Another enticing possibility is the expression of shRNA as a 3' fusion with a tRNA to allow efficient cytoplasmic delivery while supporting the eventual removal of the tRNA component. A fusion construct between tRNAVal and an shRNA with a 30 bp stem has been reported to support RNAi (K. Oshima et al. Cancer Res. 63 (2003), pp. 6809-6814), but is it unclear whether the chimeric transcript is transported intact into the cytoplasm, or is processed in the nucleus. The recent report that a predicted virus-encoded miRNA appears to be expressed as a tRNA fusion transcript (S. Pfeffer et al. Nat. Methods 2 (2005), pp. 269-276), validates the potential utility of this approach.

Due to the various limitations associated with the expression of RNAi effectors as parts of larger transcripts, the most common strategies for vector-based RNAi involve the use of self-contained RNA polymerase III promoters. shRNA and siRNA are commonly expressed from U6 (P.J. Paddison et al. Genes Dev. 16 (2002), pp. 948-958), (N.S. Lee et al. Nat. Biotechnol. 20 (2002), pp. 500-505) and (G. Sui et al. Proc. Natl. Acad. Sci. USA 99 (2002), pp. 5515-5520), H1 (T.R. Brummelkamp et al. Science 296 (2002), pp. 550-553) or 7SK (F. Czauderna et al. Nucleic Acids Res. 31 (2003), p. e127) promoters. Transcription is initiated at a precise position outside of the promoter sequence and terminates upon encountering a stretch of 4-6 thymidines in the expression cassette. Thus, for expression of an shRNA, an expression cassette encoding, in the following order, the top strand of the hairpin, the hairpin loop, the bottom strand of the hairpin, and the terminator, is inserted immediately downstream of the promoter, by various means **(****Fig. 5A****).** For expression of siRNA, two separate cassettes consisting of the promoter, the top or bottom strand and the terminator have to be generated **(****Fig. 5B****).** Previous experiences from expression of ribozymes suggest that heterogeneity in the 3' overhang of the transcribed shRNA may be generated, either through imprecise termination or the action of 3' exonucleases following termination after the fourth U in the terminator (P.D. Good et al., Gene Ther. 4 (1997), pp. 45-54). This variability is however not likely to be of significant practical importance for the efficacy of the siRNAs or shRNAs, as short 3' overhangs of variable length appear to be well tolerated within both types of molecules (S.M. Elbashir et al. EMBO J. 20 (2001), pp. 6877-6888) and (M. Miyagishi and K. Taira, Nat. Biotechnol. 20 (2002), pp. 497-500).

3.2. Practical considerations for construction of polymerase III-based RNAi vectors. Two principle methods for generating siRNA/shRNAs expression cassettes exist, each with their own advantages and drawbacks. In the first method, the cassette is generated by annealing of two complementary oligos, generating a double-stranded oligo cassette with appropriate overhangs for directional cloning downstream of the promoter. This methodology is straightforward and efficient, but the overhang sequences that are used for cloning will result in expression of an shRNA with a 5' leader sequence if the restriction site in the vector that is used for cloning is outside of the promoter. Such a leader sequence is likely to affect the potency of the transcribed siRNA. This problem can be avoided by inserting the cloning site within the 3' end of the promoter, covering positions -5 to +1 relative to the transcription start site (+1), so that the overhang from the oligo cassette will be part of the promoter proper (covering positions -4 to -1), while transcription starts at the first position in the double-stranded part of the oligo cassette, encoding the effector molecule. This strategy requires mutating the wild-type promoters to introduce a suitable cloning site. Targeted mutations within U6 and H1 promoters to introduce a BgIII site have been shown to be compatible with effective transcription and shRNA-mediated silencing of expression (M. van de Wetering et al., EMBO Rep. 4 (2003), pp. 609-615). An alternative but more cumbersome methodology, that avoids a leader sequence while retaining the wild-type promoter sequence, requires cloning of an oligo cassette with a blunt 5' end into a recessed restriction site immediately after the promoter (N.S. Lee et al. Nat. Biotechnol. 20 (2002), pp. 500-505). In addition to the oligo cassette-based cloning strategies, a PCR-based cloning strategy is also commonly employed (D. Castanotto et al. RNA 8 (2002), pp. 1454-1460). A PCR product containing the promoter and the sequences encoding the shRNA or siRNA is amplified using a 5' promoter primer in combination with a tagged 3' promoter primer in which the tag consists of the reverse complement of the expression unit. This method has the advantage that the resulting PCR products support expression of siRNA or shRNA when transfected directly into cells, and the approach is therefore useful for screening of multiple constructs for efficacy (D. Castanotto et al. RNA 8 (2002), pp. 1454-1460).

3.3. RNAi effector molecule design. Due to the sequence-dependent variability of siRNA efficacy (T. Holen et al. Nucleic Acids Res. 30 (2002), pp. 1757-1766) and (A. Khvorova et al. Cell 115 (2003), pp. 209-216), design of the effector molecules is an important factor to consider. Statistical analyses of increasingly larger groups of sequences have however resulted in the identification of design rules that substantially improve the frequency of functional siRNA (D.S. Schwarz et al. Cell 115 (2003), pp. 199-208), (A. Khvorova et al. Cell 115 (2003), pp. 209-216), (A. Reynolds et al. Nat. Biotechnol. 22 (2004), pp. 326-330) and (M. Amarzguioui and H. Prydz, Biochem. Biophys. Res. Commun. 316 (2004), pp. 1050-1058). The single most important determinant of siRNA efficacy appears to be an asymmetry in duplex end stability that mirrors that observed for naturally occurring miRNAs and which influences asymmetrical strand incorporation into RISC (M.J. Li et al., Mol. Ther. 8 (2003), pp. 196-206). Additional position-specific determinants of unknown function (A. Reynolds et al. Nat. Biotechnol. 22 (2004), pp. 326-330) and (M. Amarzguioui and H. Prydz, Biochem. Biophys. Res. Commun. 316 (2004), pp. 1050-1058), as well as target secondary structure (B.S. Heale et al. Nucleic Acids Res. 33 (2005), p. e30), also appear to contribute to overall siRNA efficacy. Although a large-scale statistical analysis of factors affecting shRNA efficacy have not yet been published, limited comparisons of siRNA and shRNA targeting the same sites suggest a similar degree of efficacy and sequence-dependence. The available evidence thus suggests that shRNA design may be based on the design rules for siRNA. In the case of U6-based expression platforms, the presence of a G at the transcription start position is highly recommended. This does, however, not represent a serious limitation, since a G in the first position of the sense strand of siRNA (the first transcribed nucleotide) is positively correlated with siRNA functionality (M. Amarzguioui and H. Prydz, Biochem. Biophys. Res. Commun. 316 (2004), pp. 1050-1058).

Most design rules for siRNA have been based on duplexes of 19 bp with 2 nt 3' overhangs, while the stems of expressed shRNAs range in size from 19 to 29 bp (T.R. Brummelkamp et al. Science 296 (2002), pp. 550-553), (J. Harborth et al. Antisense Nucleic Acid Drug Dev. 13 (2003), pp. 83-105) and (P.J. Paddison et al. Genes Dev. 16 (2002), pp. 948-958). Early reports suggested that longer stems were generally more favorable than shorter ones (P.J. Paddison et al. Genes Dev. 16 (2002), pp. 948-958). A recent report analyzing the *in vitro* Dicer processing pattern of shRNA of various stem lengths largely confirm previous conclusions (D. Siolas et al. Nat. Biotechnol. 23 (2005), pp. 227-231). The results from these studies indicate that cleavage of the duplex occurs in a precise manner 21-22 nt from the open end of the stem. Hairpins of different lengths with extension of the duplex towards the loop would thus be expected to generate the same processed product, and any differences in efficacy between the precursor hairpins should reflect differences in either Dicer-mediated processing or cytoplasmic transport of the precursor. While differential transport efficiency cannot be discounted, the recent observations that asymmetrical siRNA of extended stems display similar improvements in efficacy as shRNA (D.H. Kim et al. Nat. Biotechnol. 23 (2005), pp. 222-226) and (S.D. Rose et al. Nucleic Acid Res. 33 (2005), pp. 4140-4156), suggest that enhanced Dicer-mediated processing contributes chiefly to this improvement. Furthermore, Dicer processing appears to also confer an asymmetry in strand incorporation into RISC, as the strand bearing the 3' overhang (the bottom strand in an expressed hairpin) is utilized preferentially (S.D. Rose et al. Nucleic Acid Res. 33 (2005), pp. 4140-4156). The above combined data thus suggest that shRNA design should proceed according to the following steps:
1. Select the desired 21 mer siRNA sequence using the most current design rules.
2. Extend the above sequence towards the 3' end of the target, for a duplex length of 25-29 bp.
3. Cap off the 3' end of the duplex (the bottom strand being the guide strand) with a loop, preferably one derived from a naturally occurring miRNA.

3.4. Inducible expression of RNAi effectors. Constitutive knockdown of gene expression can be circumvented through inducible expression of the RNAi effector molecules. The last several years has seen a rapid development of various methodologies for inducible expression of shRNAs from polymerase III promoters. The first method to be described was based on the tetracycline-inducible system (M. Gossen et al. Science 268 (1995), pp. 1766-1769). A tetracycline-inducible H1 promoter was generated by replacement of a 19 bp sequence between the TATA box and the transcription start site with a binding site (tetO) for the tetracycline repressor (M. van de Wetering et al., EMBO Rep. 4 (2003), pp. 609-615). In transgenic cells expressing the repressor, repressor binding to the tetO site blocks transcription, while addition of the inducer tetracycline or its derivative, doxycycline, results in dissociation of the repressor, allowing transcription to proceed. A similar strategy has been employed to generate Tet-responsive U6 (F. Czauderna et al., Nucleic Acids Res. 31 (2003), p. e127), (S. Matsukura et al. Nucleic Acids Res. 31 (2003), p. e77) and (X. Lin et al. FEBS Lett. 577 (2004), pp. 376-380) and 7SK (F. Czauderna et al., Nucleic Acids Res. 31 (2003), p. e127) promoter based silencing vectors. Furthermore, replacement of a 26-nt sequence between the TATA box and the transcription start site in the H1 promoter with a lac operator, results in IPTG-responsive shRNA expression in transgenic cells expressing the lac repressor (M. Higuchi et al. Cancer Sci. 95 (2004), pp. 442-447).

The tet-responsive polymerase III promoters display some level of leakiness, which may result in significant downregulation even in the absence of induction when working with very potent shRNA (X. Lin et al. FEBS Lett. 577 (2004), pp. 376-380). A more tightly regulated U6 promoter was recently described. This expression system contains two optimally placed tet operators and displays a combination of low basal transcriptional activity and effective silencing in the induced state (X. Lin et al. FEBS Lett. 577 (2004), pp. 376-380). An alternative to the tetracycline-inducible system is the more tightly regulated but generally less active ecdysone-inducible system (D. No et al. Proc. Natl. Acad. Sci. USA 93 (1996), pp. 3346-3351). Ecdysone (muristerone A)-inducible expression of shRNA under a modified U6 promoter, in which the U6 enhancer was replaced with a GAL4 element, has been shown to facilitate efficient inducible silencing of target gene expression in cells expressing a GAL4-Oct-2^{Q} transactivator fusion and the nuclear receptor/transcription factors VgEcR and RXR (S. Gupta et al. Proc. Natl. Acad. Sci. USA 101 (2004), pp. 1927-1932). Addition of the ecdysone analogue initiates an activation cascade involving dimerization of the transgenic transcription factors, activation of GAL4-Oct-2^{Q} transactivator expression, and finally, activation of the modified U6 promoter.

3.5. Tissue-specific transcription. The use of RNA polymerase II (pol II) promoters is essential for tissue-specific transcription. Such transcripts obtained from the pol II promoter are, in most cases, long dsRNA that, when transferred to the cytosol, induce interferon response. However, deviation from the strict double-stranded nature of the transcript, such as introduction of a short hairpin or bulge, might prevent interferon stimulation, as seems to be the case for primary miRNA transcripts (pri-miRNAs). An alternative mechanism, which could avoid interferon stimulation is the transcription of RNAs that lack the necessary signals for transport to the cytoplasm. Thus, a vector has been developed to express transcripts from pol II promoters that lack the 5'-cap structure and the 3'-poly(A) tail, which are responsible for the export from the nucleus; thus the interferon response is not induced (Shinagawa, T and Ishii S. 2003 Genes Dev 17:1340-1345). In their method, Shinagawa and Ishii first expressed long double-stranded RNAs (dsRNAs) from a CMV (pol II) promoter. In order to abolish capping, a cis-acting ribozyme coding region was cloned downstream of the hairpin dsRNA template. Poly(A) addition was prevented by addition of a pol II transcriptional pause site (and not a poly(A) addition site) downstream of the dsRNA template. Additionally, functional siRNAs have been expressed in cell lines from expression units containing a CMV promoter and a minimal poly(A) cassette, which presumably avoids poly(A) tailing (Xia, H. et al. 2002 Nature Biotech 20:1006-1010).

### Administration of Nucleic Acid Molecules to Host Cells

A siRNA molecule can be adapted for use to treat, for example, variety of disease and conditions described herein, such as proliferative diseases and conditions and/or cancer including breast cancer, cancers of the head and neck including various lymphomas such as mantle cell lymphoma, non-Hodgkins lymphoma, adenoma, squamous cell carcinoma, laryngeal carcinoma, cancers of the retina, cancers of the esophagus, multiple myeloma, ovarian cancer, uterine cancer, melanoma, colorectal cancer, bladder cancer, prostate cancer, glioblastoma, lung cancer (including non-small cell lung carcinoma), pancreatic cancer, cervical cancer, head and neck cancer, skin cancers, nasopharyngeal carcinoma, liposarcoma, epithelial carcinoma, renal cell carcinoma, gallbladder adeno carcinoma, parotid adenocarcinoma, endometrial sarcoma, and multidrug resistant cancers. For example, a siNA molecule can comprise a delivery vehicle, including liposomes, for administration to a subject, carriers and diluents and their salts, and/or can be present in pharmaceutically acceptable formulations. Methods for the delivery of nucleic acid molecules are described in Akhtar et al., 1992 Trends Cell Bio 2:139. These protocols can be utilized for the delivery of virtually any nucleic acid molecule. Nucleic acid molecules can be administered to cells by a variety of methods known to those of skill in the art, including, but not restricted to, encapsulation in liposomes, by iontophoresis or by incorporation into other vehicles, such as biodegradable polymers, hydrogels, cyclodextrins, PLGA and PLCA microspheres, biodegradable nanocapsules, and bioadhesive microspheres, or by proteinaceous vectors. The nucleic acid molecules can also be formulated or complexed with polyethyleneimine and derivatives thereof, such as polyethyleneimine-polyethyleneglycol-N-acetylgalactosamine (PEI-PEG-GAL) or polyethyleneimine-polyethyleneglycol-tri-N-acetylgalactosamine (PEI-PEG-triGAL) derivatives. Alternatively, the nucleic acid/vehicle combination is locally delivered by direct injection or by use of an infusion pump.

siRNA molecule is can be designed or formulated to specifically target tumor cells. For example, various formulations and conjugates can be utilized to specifically target tumor cells, including PEI-PEG-folate, PEI-PEG-RGD, PEI-PEG-biotin, PEI-PEG-cholesterol, and other conjugates known in the art that enable specific targeting to tumor cells

The nucleic acid molecules can be administered via pulmonary delivery, such as by inhalation of an aerosol or spray dried formulation administered by an inhalation device or nebulizer, providing rapid local uptake of the nucleic acid molecules into relevant pulmonary tissues. Solid particulate compositions containing respirable dry particles of micronized nucleic acid compositions can be prepared by grinding dried or lyophilized nucleic acid compositions, and then passing the micronized composition through, for example, a 400 mesh screen to break up or separate out large agglomerates. A solid particulate composition comprising the nucleic acid compositions can optionally contain a dispersant which serves to facilitate the formation of an aerosol as well as other therapeutic compounds. A suitable dispersant is lactose, which can be blended with the nucleic acid compound in any suitable ratio, such as a 1 to 1 ratio by weight. Aerosols of liquid particles comprising a nucleic acid composition can be produced by any suitable means, such as with a nebulizer. Nebulizers are commercially available devices which transform solutions or suspensions of an active ingredient into a therapeutic aerosol mist either by means of acceleration of a compressed gas, typically air or oxygen, through a narrow venturi orifice or by means of ultrasonic agitation. Suitable formulations for use in nebulizers comprise the active ingredient in a liquid carrier in an amount of up to 40% w/w preferably less than 20% w/w of the formulation. The carrier is typically water or a dilute aqueous alcoholic solution, preferably made isotonic with body fluids by the addition of, for example, sodium chloride or other suitable salts. Optional additives include preservatives if the formulation is not prepared sterile, for example, methyl hydroxybenzoate, anti-oxidants, flavorings, volatile oils, buffering agents and emulsifiers and other formulation surfactants. The aerosols of solid particles comprising the active composition and surfactant can likewise be produced with any solid particulate aerosol generator. Aerosol generators for administering solid particulate therapeutics to a subject produce particles which are respirable, as explained above, and generate a volume of aerosol containing a predetermined metered dose of a therapeutic composition at a rate suitable for human administration. One illustrative type of solid particulate aerosol generator is an insufflator. Suitable formulations for administration by insufflation include finely comminuted powders which can be delivered by means of an insufflator. In the insufflator, the powder, e.g., a metered dose thereof effective to carry out the treatments described herein, is contained in capsules or cartridges, typically made of gelatin or plastic, which are either pierced or opened in situ and the powder delivered by air drawn through the device upon inhalation or by means of a manually-operated pump. The powder employed in the insufflator consists either solely of the active ingredient or of a powder blend comprising the active ingredient, a suitable powder diluent, such as lactose, and an optional surfactant. The active ingredient typically comprises from 0.1 to 100 w/w of the formulation. A second type of illustrative aerosol generator comprises a metered dose inhaler. Metered dose inhalers are pressurized aerosol dispensers, typically containing a suspension or solution formulation of the active ingredient in a liquified propellant. During use these devices discharge the formulation through a valve adapted to deliver a metered volume to produce a fine particle spray containing the active ingredient. Suitable propellants include certain chlorofluorocarbon compounds, for example, dichlorodifluoromethane, trichlorofluoromethane, dichlorotetrafluoroethane and mixtures thereof. The formulation can additionally contain one or more co-solvents, for example, ethanol, emulsifiers and other formulation surfactants, such as oleic acid or sorbitan trioleate, anti-oxidants and suitable flavoring agents.

A siRNA molecule can be complexed with membrane disruptive agents. The membrane disruptive agent or agents and the siRNA molecule may also complexed with a cationic lipid or helper lipid molecule.

Thus, described is a pharmaceutical composition comprising one or more nucleic acid(s) in an acceptable carrier, such as a stabilizer, buffer, and the like. The polynucleotides can be administered (e.g., RNA, DNA) and introduced into a subject by any standard means, with or without stabilizers, buffers, and the like, to form a pharmaceutical composition. When it is desired to use a liposome delivery mechanism, standard protocols for formation of liposomes can be followed. The compositions can also be formulated and used as tablets, capsules or elixirs for oral administration, suppositories for rectal administration, sterile solutions, suspensions for injectable administration, and the other compositions known in the art.

Further described are pharmaceutically acceptable formulations of the compounds described. These formulations include salts of the above compounds, e.g., acid addition salts, for example, salts of hydrochloric, hydrobromic, acetic acid, and benzene sulfonic acid.

A pharmacological composition or formulation refers to a composition or formulation in a form suitable for administration, *e.g*., systemic administration, into a cell or subject, including for example a human. Suitable forms, in part, depend upon the use or the route of entry, for example oral, transdermal, or by injection. Such forms should not prevent the composition or formulation from reaching a target cell (*i.e.,* a cell to which the negatively charged nucleic acid is desirable for delivery). For example, pharmacological compositions injected into the blood stream should be soluble. Other factors are known in the art, and include considerations such as toxicity and forms that prevent the composition or formulation from exerting its effect.

By "systemic administration" is meant *in vivo* systemic absorption or accumulation of drugs in the blood stream followed by distribution throughout the entire body. Administration routes that lead to systemic absorption include, without limitation: intravenous, subcutaneous, intraperitoneal, inhalation, oral, intrapulmonary and intramuscular. Each of these administration routes exposes the siRNA molecules to an accessible diseased tissue. The rate of entry of a drug into the circulation has been shown to be a function of molecular weight or size. The use of a liposome or other drug carrier comprising the compounds can potentially localize the drug, for example, in certain tissue types, such as the tissues of the reticular endothelial system (RES). A liposome formulation that can facilitate the association of drug with the surface of cells, such as, lymphocytes and macrophages is also useful. This approach can provide enhanced delivery of the drug to target cells by taking advantage of the specificity of macrophage and lymphocyte immune recognition of abnormal cells.

By "pharmaceutically acceptable formulation" is meant, a composition or formulation that allows for the effective distribution of the nucleic acid molecules in the physical location most suitable for their desired activity. Non-limiting examples of agents suitable for formulation with the nucleic acid molecules of the instant invention include: P-glycoprotein inhibitors (such as Pluronic P85), biodegradable polymers, such as poly (DL-lactide-coglycolide) microspheres for sustained release delivery, and loaded nanoparticles, such as those made of polybutylcyanoacrylate.

Further described is the use of the composition comprising surface-modified liposomes containing poly (ethylene glycol) lipids (PEG-modified, or long-circulating liposomes or stealth liposomes). These formulations offer a method for increasing the accumulation of drugs in target tissues. This class of drug carriers resists opsonization and elimination by the mononuclear phagocytic system (MPS or RES), thereby enabling longer blood circulation times and enhanced tissue exposure for the encapsulated drug. Such liposomes have been shown to accumulate selectively in tumors, presumably by extravasation and capture in the neovascularized target tissues. The long-circulating liposomes enhance the pharmacokinetics and pharmacodynamics of DNA and RNA, particularly compared to conventional cationic liposomes which are known to accumulate in tissues of the MPS. Long-circulating liposomes are also likely to protect drugs from nuclease degradation to a greater extent compared to cationic liposomes, based on their ability to avoid accumulation in metabolically aggressive MPS tissues such as the liver and spleen.

Further described are compositions prepared for storage or administration that include a pharmaceutically effective amount of the desired compounds in a pharmaceutically acceptable carrier or diluent. Acceptable carriers or diluents for therapeutic use are well known in the pharmaceutical art, and are described, for example, in Remington's Pharmaceutical Sciences, Mack Publishing Co. (A. R. Gennaro edit. 1985). For example, preservatives, stabilizers, dyes and flavoring agents can be provided. These include sodium benzoate, sorbic acid and esters of p-hydroxybenzoic acid. In addition, antioxidants and suspending agents can be used.

A pharmaceutically effective dose is that dose required to prevent, inhibit the occurrence, or treat (alleviate a symptom to some extent, preferably all of the symptoms) of a disease state. The pharmaceutically effective dose depends on the type of disease, the composition used, the route of administration, the type of mammal being treated, the physical characteristics of the specific mammal under consideration, concurrent medication, and other factors that those skilled in the medical arts will recognize. Generally, an amount between 0.1 mg/kg and 100 mg/kg body weight/day of active ingredients is administered dependent upon potency of the negatively charged polymer.

The nucleic acid molecules and formulations thereof can be administered orally, topically, parenterally, by inhalation or spray, or rectally in dosage unit formulations containing conventional non-toxic pharmaceutically acceptable carriers, adjuvants and/or vehicles. The term parenteral as used herein includes percutaneous, subcutaneous, intravascular (*e.g*., intravenous), intramuscular, or intrathecal injection or infusion techniques and the like. In addition, there is provided a pharmaceutical formulation comprising a nucleic acid molecule of the invention and a pharmaceutically acceptable carrier. One or more nucleic acid molecules can be present in association with one or more non-toxic pharmaceutically acceptable carriers and/or diluents and/or adjuvants, and if desired other active ingredients. The pharmaceutical compositions containing nucleic acid molecules can be in a form suitable for oral use, for example, as tablets, troches, lozenges, aqueous or oily suspensions, dispersible powders or granules, emulsion, hard or soft capsules, or syrups or elixirs.

Nucleic acid molecules can be administered parenterally in a sterile medium. The drug, depending on the vehicle and concentration used, can either be suspended or dissolved in the vehicle. Advantageously, adjuvants such as local anesthetics, preservatives and buffering agents can be dissolved in the vehicle.

Dosage levels of the order of from about 0.1 mg to about 140 mg per kilogram of body weight per day are useful in the treatment of the above-indicated conditions (about 0.5 mg to about 7 g per subject per day). The amount of active ingredient that can be combined with the carrier materials to produce a single dosage form varies depending upon the host treated and the particular mode of administration. Dosage unit forms generally contain between from about 1 mg to about 500 mg of an active ingredient.

It is understood that the specific dose level for any particular subject depends upon a variety of factors including the activity of the specific compound employed, the age, body weight, general health, sex, diet, time of administration, route of administration, and rate of excretion, drug combination and the severity of the particular disease undergoing therapy.

The nucleic acid molecules can also be administered to a subject in combination with other therapeutic compounds to increase the overall therapeutic effect. The use of multiple compounds to treat an indication can increase the beneficial effects while reducing the presence of side effects.

Further described are compositions suitable for administering nucleic acid molecules of the invention to specific cell types. For example, the asialoglycoprotein receptor (ASGPr) is unique to hepatocytes and binds branched galactose-terminal glycoproteins, such as asialoorosomucoid (ASOR). In another example, the folate receptor is overexpressed in many cancer cells. Binding of such glycoproteins, synthetic glycoconjugates, or folates to the receptor takes place with an affinity that strongly depends on the degree of branching of the oligosaccharide chain, for example, triatennary structures are bound with greater affinity than biatenarry or monoatennary chains. Investigators obtained this high specificity through the use of N-acetyl-D-galactosamine as the carbohydrate moiety, which has higher affinity for the receptor, compared to galactose. This "clustering effect" has also been described for the binding and uptake of mannosyl-terminating glycoproteins or glycoconjugates. The use of galactose, galactosamine, or folate based conjugates to transport exogenous compounds across cell membranes can provide a targeted delivery approach to, for example, the treatment of liver disease, cancers of the liver, or other cancers. The use of bioconjugates can also provide a reduction in the required dose of therapeutic compounds required for treatment. Furthermore, therapeutic bioavialability, pharmacodynamics, and pharmacokinetic parameters can be modulated through the use of nucleic acid bioconjugates of the invention. Nucleic acid molecules can be complexed with or covalently attached to nanoparticles, such as Hepatitis B virus S, M, or L envelope proteins. Nucleic acid molecules can be delivered with specificity for human tumor cells, specifically non-apoptotic human tumor cells including for example T-cells, hepatocytes, breast carcinoma cells, ovarian carcinoma cells, melanoma cells, intestinal epithelial cells, prostate cells, testicular cells, non-small cell lung cancers, small cell lung cancers, etc.

Alternatively, certain siRNA molecules can be expressed within cells from eukaryotic promoters. Those skilled in the art realize that any nucleic acid can be expressed in eukaryotic cells from the appropriate DNA/RNA vector. The activity of such nucleic acids can be augmented by their release from the primary transcript by a enzymatic nucleic acid.

RNA molecules can be expressed from transcription units inserted into DNA or RNA vectors. The recombinant vectors can be DNA plasmids or viral vectors. siRNA expressing viral vectors can be constructed based on, but not limited to, adeno-associated virus, retrovirus, adenovirus, or alphavirus. In another embodiment, pol III based constructs are used to express nucleic acid molecules of the invention. The recombinant vectors capable of expressing the siRNA molecules can be delivered as described above, and persist in target cells. Alternatively, viral vectors can be used that provide for transient expression of nucleic acid molecules. Such vectors can be repeatedly administered as necessary. Once expressed, the siRNA molecule interacts with the target mRNA and generates an RNAi response. Delivery of siRNA molecule expressing vectors can be systemic, such as by intravenous or intramuscular administration, by administration to target cells ex-planted from a subject followed by reintroduction into the subject, or by any other means that would allow for introduction into the desired target cell.

Further described is an expression vector comprising a nucleic acid sequence encoding at least one siRNA molecule. The expression vector can encode one or both strands of a siRNA duplex, or a single self-complementary strand that self hybridizes into a siRNA duplex. The nucleic acid sequences encoding the siRNA molecules can be operably linked in a manner that allows expression of the siNA molecule.

Further described is an expression vector comprising: a) a transcription initiation region (*e.g.*, eukaryotic pol I, II or III initiation region); b) a transcription termination region (e.g., eukaryotic pol I, II or III termination region); and c) a nucleic acid sequence encoding at least one of the siRNA molecules, wherein said sequence is operably linked to said initiation region and said termination region in a manner that allows expression and/or delivery of the siRNA molecule.

Transcription of the siRNA molecule sequences can be driven from a promoter for eukaryotic RNA polymerase I (pol I), RNA polymerase II (pol II), or RNA polymerase III (pol III). Transcripts from pol II or pol III promoters are expressed at high levels in all cells; the levels of a given pol II promoter in a given cell type depends on the nature of the gene regulatory sequences (enhancers, silencers, etc.) present nearby. Prokaryotic RNA polymerase promoters are also used, providing that the prokaryotic RNA polymerase enzyme is expressed in the appropriate cells. Several investigators have demonstrated that nucleic acid molecules expressed from such promoters can function in mammalian cells. More specifically, transcription units such as the ones derived from genes encoding U6 small nuclear (snRNA), transfer RNA (tRNA) and adenovirus VA RNA are useful in generating high concentrations of desired RNA molecules such as siRNA in cells. The above siRNA transcription units can be incorporated into a variety of vectors for introduction into mammalian cells, including but not restricted to, plasmid DNA vectors, viral DNA vectors (such as adenovirus or adeno-associated virus vectors), or viral RNA vectors (such as retroviral or alphavirus vectors).

### Methods for monitoring efficacy of the siRNA

The consequences of inhibition can be confirmed by examination of the outward properties of the cell or organism or infectious agent or by biochemical techniques such as RNA solution hybridization, nuclease protection, Northern hybridization, reverse transcription, gene expression monitoring with a microarray, antibody binding, enzyme linked immunosorbent assay (ELISA), Western blotting, radioimmunoassay (RIA), other immunoassays, and fluorescence activated cell analysis (FACS). For RNA-mediated inhibition in a cell line or whole organism, gene expression is conveniently assayed by use of a reporter or drug resistance gene whose protein product is easily assayed.

The present invention overcomes deficiencies in the prior art by describing antisense nucleic acids compositions comprising a domain. The domain is designed to hybridize to complementary nucleic acid target domains in a target RNA, and inhibit translation, processing, transport, or binding by proteins or riboproteins. The target domain is limited to a poly A tail. Target domains exclude, AUG, 5' non- translated sequences, translation initiation factor binding sites, ribosome subunit binding sites, Shine Dalgarno sequence, 3' nontranslated sequences, poly-addition site, 3' cleavage sites, coding regions, introns, intron branch sites, intron/exon junctions, and splice sequences. Target RNA may be human. Methods for using these antisense compositions include inhibiting translation of an RNA and treating cancer in a human. Therapeutic antisense compositions are also described.

The following section outlines existing antisense technology, including antisense transgene construction, viral and non-viral delivery and expression vectors, and antisense oligonucleotides. Descriptions of examples are given for target domains and target genes as well as methods for administering antisense compositions and disease treatment.

### I. Antisense

Antisense methodology takes advantage of the fact that nucleic acids tend to pair with "complementary" sequences. The term "complementary" is intended to refer to polynucleotides that are capable of base-pairing according to the standard Watson-Crick complementarity rules. That is, the larger purines will base pair with the smaller pyrimidines to form combinations of guanine paired with cytosine (G:C) and either adenine paired with thymine (A:T) in the case of DNA, or adenine paired with uracil (A:U) in the case of RNA. Inclusion of less common bases such as inosine, 5-methylcytosine, 6-methyladenine, hypoxanthine *et al.* in hybridizing sequences does not interfere with pairing.

Targeting double-stranded (ds) DNA with polynucleotides leads to triple-helix formation; targeting RNA will lead to double-helix formation. Antisense construct polynucleotides, when introduced into a target cell, specifically bind to their target polynucleotide and interfere with transcription, RNA processing, transport, translation and/or stability. Antisense RNA constructs, or DNA encoding such antisense RNAs, may be employed to inhibit gene transcription, translation, or both within a host cell, either in *vitro* or *in vivo,* such as within a host animal, including a human subject.

Antisense constructs may be designed to bind to the poly A tail. It is contemplated that the most effective antisense constructs will include regions complementary to the poly A tail. The amount of poly A tail material included will vary depending on the particulars and circumstances. One can readily test whether too much material is included simply by testing the constructs *in vitro* to determine whether cellular function is affected or whether the expression of genes having complementary sequences is affected.

As stated above, "complementary" or "antisense" means polynucleotide sequences that are substantially complementary over their entire length and have very few base mismatches. For example, sequences of fifteen bases in length may be termed complementary when they have complementary nucleotides at thirteen or fourteen positions. Naturally, sequences that are completely complementary will be sequences that are entirely complementary throughout their entire length and have no base mismatches. Other sequences with lower degrees of homology are also contemplated. For example, an antisense construct that has limited regions of high homology, but also contains a non-homologous region (e.g., ribozyme) could be designed. These molecules, though having less than 50% homology, would bind to target sequences under appropriate conditions.

It may be advantageous to combine portions of genomic DNA with cDNA or synthetic sequences to generate specific constructs. For example, a genomic clone may be convenient to be used. The cDNA or a synthesized polynucleotide may provide more convenient restriction sites for the remaining portion of the construct and, therefore, would be used for the rest of the sequence.

### A. Antisense Transgenes

Within certain embodiments expression vectors are used in therapeutic applications. Expression requires that appropriate signals be provided in the vectors, which include various regulatory elements, such as enhancers/promoters from both viral and mammalian sources that drive expression of the genes of interest in host cells. Elements designed to optimize messenger RNA stability and translatability in host cells are also defined. The conditions for the use of a number of dominant drug selection markers for establishing permanent, stable cell clones expressing the products are also provided, as is an element that links expression of the drug selection markers to expression of the polypeptide.

Throughout this application, the term "expression construct" is meant to include any type of genetic construct containing a polynucleotide coding for a gene product in which part or all of the polynucleotide encoding sequence is capable of being transcribed. The transcript may be translated into a protein, but it need not be. The term expression includes both transcription of a gene and translation of mRNA into a gene product. Preferably, the term expression only includes transcription of the polynucleotide encoding a gene of interest.

In preferred embodiments, the polynucleotide encoding a gene product is under transcriptional control of a promoter. A "promoter" refers to a DNA sequence recognized by the synthetic machinery of the cell, or introduced synthetic machinery, required to initiate the specific transcription of a gene. The phrase "under transcriptional control" means that the promoter is in the correct location and orientation in relation to the polynucleotide to control RNA polymerase initiation and expression of the gene.

The term promoter will be used here to refer to a group of transcriptional control modules that are clustered around the initiation site for RNA polymerase II. Much of the thinking about how promoters are organized derives from analyses of several viral promoters, including those for the HSV thymidine kinase (tk) and SV40 early transcription units. These studies, augmented by more recent work, have shown that promoters are composed of discrete functional modules, each consisting of approximately 7-20 bp of DNA, and containing one or more recognition sites for transcriptional activator or repressor proteins.

At least one module in each promoter functions to position the start site for RNA synthesis. The best known example of this is the TATA box, but in some promoters lacking a TATA box, such as the promoter for the mammalian terminal deoxynucleotidyl transferase gene and the promoter for the SV40 late genes, a discrete element overlying the start site itself helps to fix the place of initiation.

Additional promoter elements regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the tk promoter, the spacing between promoter elements can be increased to 50 bp apart before activity begins to decline. Depending on the promoter, it appears that individual elements can function either co-operatively or independently to activate transcription.

The particular promoter employed to control the expression of a polynucleotide sequence of interest is not believed to be important, so long as it is capable of directing the expression of the polynucleotide in the targeted cell. Thus, where a human cell is targeted, it is preferable to position the polynucleotide coding region adjacent to and under the control of a promoter that is capable of being expressed in a human cell. Generally speaking, such a promoter might include either a human or viral promoter.

The human cytomegalovirus (CMV) immediate early gene promoter, the SV40 early promoter, the Rous sarcoma virus long terminal repeat, rat insulin promoter and glyceraldehyde-3-phosphate dehydrogenase can be used to obtain high-level expression of the coding sequence of interest. The use of other viral or mammalian cellular or bacterial phage promoters which are well-known in the art to achieve expression of a sequence of interest is contemplated as well, provided that the levels of expression are sufficient for a given purpose.

Selection of a promoter that is regulated in response to specific physiologic or synthetic signals can permit inducible expression of the gene product. For example in the case where expression of a transgene, or transgenes when a multicistronic vector is utilized, is toxic to the cells in which the vector is produced in, it may be desirable to prohibit or reduce expression of one or more of the transgenes. Examples of transgenes that may be toxic to the producer cell line are pro-apoptotic and cytokine genes. Several inducible promoter systems are available for production of viral vectors where the transgene product may be toxic.

The Ecdysone-Inducible Mammalian Expression System (Invitrogen, Carlsbad, CA) is one such system. This system is designed to allow regulated expression of a gene of interest in mammalian cells. It consists of a tightly regulated expression mechanism that allows virtually no basal level expression of the transgene, but over 200-fold inducibility. The system is based on the heterodimeric ecdysone receptor of *Drosophila,* and when ecdysone or an analog such as muristerone A binds to the receptor, the receptor activates a promoter to turn on expression of the downstream transgene high levels of mRNA transcripts are attained. In this system, both monomers of the heterodimeric receptor are constitutively expressed from one vector, whereas the ecdysone-responsive promoter which drives expression of the gene of interest is on another plasmid. Engineering of this type of system into the gene transfer vector of interest would therefore be useful. Cotransfection of plasmids containing the gene of interest and the receptor monomers in the producer cell line would then allow for the production of the gene transfer vector without expression of a potentially toxic transgene. At the appropriate time, expression of the transgene could be activated with ecdysone or muristeron A.

Another inducible system that would be useful is the Tet-Off™ or Tet-On™ system (Clontech, Palo Alto, CA) originally developed by Gossen and Bujard. This system also allows high levels of gene expression to be regulated in response to tetracycline or tetracycline derivatives such as doxycycline. In the Tet-On™ system, gene expression is turned on in the presence of doxycycline, whereas in the Tel-Off™ system, gene expression is turned on in the absence of doxycycline. These systems are based on two regulatory elements derived from the tetracycline resistance operon of *E*. *coli.* The gene of interest is cloned into a plasmid behind a promoter that has tetracycline- responsive elements present in it. A second plasmid contains a regulatory element called the tetracycline-controlled transactivator, which is composed, in the Tet-Off™ system, of the VP16 domain from the herpes simplex virus and the wild-type tertracycline repressor. Thus in the absence of doxycycline, transcription is constitutively on. In the Tet-On™ system, the tetracycline repressor is not wild type and in the presence of doxycycline activates transcription. For gene therapy vector production, the Tet-Off™ system would be preferable so that the producer cells could be grown in the presence of tetracycline or doxycycline and prevent expression of a potentially toxic transgene, but when the vector is introduced to the patient, the gene expression would be constitutively on.

An inducible system particularly useful with the present invention is a radiation-inducible system. Ionizing radiation-inducible promoters include a CArG domain of an *Ego-1* promoter, a *los* promoter, a c-jun promoter, and a TNF-α promoter, which can be operatively linked to a protein expression region. In this regard, U.S. Patent 5,612,318, is instructive.

In some circumstances, it may be desirable to regulate expression of a transgene in a gene therapy vector. For example, different viral promoters with varying strengths of activity may be utilized depending on the level of expression desired. In mammalian cells, the CMV immediate early promoter if often used to provide strong transcriptional activation. Modified versions of the CMV promoter that are less potent have also been used when reduced levels of expression of the transgene are desired. When expression of a transgene in hematopoetic cells is desired, retroviral promoters such as the LTRs from MLV or MMTV are often used. Other viral promoters that may be used depending on the desired effect include SV40, RSV LTR, HIV-1 and HIV-2 LTR, adenovirus promoters such as from the E1A, E2A, or MLP region, AAV LTR, cauliflower mosaic virus, HSV-TK, and avian sarcoma virus.

Similarly tissue specific promoters may be used to effect transcription in specific tissues or cells so as to reduce potential toxicity or undesirable effects to non- targeted tissues. For example, promoters such as the PSA, probasin, prostatic acid phosphatase or prostate-specific glandular kallikrein (hK2) may be used to target gene expression in the prostate.

In certain indications, it may be desirable to activate transcription at specific times after administration of the gene therapy vector. This may be done with such promoters as those that are hormone or cytokine regulatable. For example in gene therapy applications where the indication is a gonadal tissue where specific steroids are produced or routed to, use of androgen or estrogen regulated promoters may be advantageous. Such promoters that are hormone regulatable include MMTV, MT-1, ecdysone and RuBisco. Other hormone regulated promoters such as those responsive to thyroid, pituitary and adrenal hormones are expected to be useful in the present invention. Cytokine and inflammatory protein responsive promoters that could be used include K and T Kininogen, c-fos, TNF-alpha, C-reactive protein, haptoglobin, serum amyloid A2, C/EBP alpha, IL-1, IL-6, Complement C3, IL-8, alpha-1 acid glycoprotein, alpha-1 antitypsin, lipoprotein lipase, angiotensinogen, fibrinogen, c-jun (inducible by phorbol esters, TNF-alpha, UV radiation, retinoic acid, and hydrogen peroxide), collagenase (induced by phorbol esters and retinoic acid), metallothionein (heavy metal and glucocorticoid inducible), Stromelysin (inducible by phorbol ester, interleukin-1 and EGF), alpha-2 macroglobulin and alpha-1 antichymotrypsin.

Tumor specific promoters such as osteocalcin, hypoxia-responsive element (HRE), MAGE-4, CEA, alpha-fetoprotein, GRP78/BiP and tyrosinase may also be used to regulate gene expression in tumor cells. Other promoters that could be used according to the present invention include Lac-regulatable, chemotherapy inducible (*e.g*. MDR), and heat (hyperthermia) inducible promoters, radiation-inducible (*e.g*., EGR), Alpha-inhibin, RNA pol III tRNA met and other amino acid promoters, U1 snRNA, MC-1, PGK, β-actin and α-globin. Many other promoters may also be useful.

Enhancers are genetic elements that increase transcription from a promoter located at a distant position on the same molecule of DNA. Enhancers are organized much like promoters. That is, they are composed of many individual elements, each of which binds to one or more transcriptional proteins. The basic distinction between enhancers and promoters is operational. An enhancer region as a whole must be able to stimulate transcription at a distance; this need not be true of a promoter region or its component elements. On the other hand, a promoter must have one or more elements that direct initiation of RNA synthesis at a particular site and in a particular orientation, whereas enhancers lack these specificities. Promoters and enhancers are frequently overlapping and contiguous, often seeming to have a very similar modular organization.

Where a cDNA insert is employed, one will typically desire to include a polyadenylation signal to effect proper polyadenylation of the gene transcript. The nature of the polyadenylation signal is not believed to be crucial to the successful practice and any such sequence may be employed such as human growth hormone and SV40 polyadenylation signals. Also contemplated as an element of the expression cassette is a terminator. These elements can serve to enhance message levels and to minimize read through from the cassette into other sequences.

### B. Viral Expression Vectors

There are a number of ways to introduce expression vectors into cells. In certain embodiments of the invention, the expression construct comprises a virus or engineered construct derived from a viral genome. The ability of certain viruses to enter cells via receptor-mediated endocytosis, to integrate into host cell genome and express viral genes stably and efficiently have made them attractive candidates for the transfer of foreign genes into mammalian cells. The first viruses used as gene vectors were DNA viruses including the papovaviruses (simian virus 40, bovine papilloma virus, and polyoma). These have a relatively low capacity for foreign DNA sequences and have a restricted host spectrum. Furthermore, their oncogenic potential and cytopathic effects in permissive cells raise safety concerns. They can accommodate only up to 8 kb of foreign genetic material but can be readily introduced in a variety of cell lines and laboratory animals.

One of the preferred methods for *in vivo* delivery involves the use of an adenovirus expression vector. "Adenovirus expression vector" is meant to include those constructs containing adenovirus sequences sufficient to (a) support packaging of the construct and (b) to express an antisense polynucleotide that has been cloned therein. In this context, expression does not require that the gene product be synthesized.

The expression vector comprises a genetically engineered form of adenovirus. Knowledge of the genetic organization of adenovirus, a 36 kb, linear, double-stranded DNA virus, allows substitution of large pieces of adenoviral DNA with foreign sequences up to 7 kb. In contrast to retrovirus, the adenoviral infection of host cells does not result in chromosomal integration because adenoviral DNA can replicate in an episomal manner without potential genotoxicity. Also, adenoviruses are structurally stable, and no genome rearrangement has been detected after extensive amplification. Adenovirus can infect virtually all epithelial cells regardless of their cell cycle stage. So far, adenoviral infection appears to be linked only to mild disease such as acute respiratory disease in humans.

Adenovirus is particularly suitable for use as a gene transfer vector because of its mid-sized genome, ease of manipulation, high titer, wide target cell range and high infectivity. Both ends of the viral genome contain 100-200 base pair inverted repeats (ITRs), which are *cis* elements necessary for viral DNA replication and packaging. The early (E) and late (L) regions of the genome contain different transcription units that are divided by the onset of viral DNA replication. The E1 region (El A and E1B) encodes proteins responsible for the regulation of transcription of the viral genome and a few cellular genes. The expression of the E2 region (E2A and E2B) results in the synthesis of the proteins for viral DNA replication. These proteins are involved in DNA replication, late gene expression and host cell shut-off. The products of the late genes, including the majority of the viral capsid proteins, are expressed only after significant processing of a single primary transcript issued by the major late promoter (MLP). The MLP, (located at 16.8 m.u.) is particularly efficient during the late phase of infection, and all the mRNAs issued from this promoter possess a 5'-tripartite leader (TPL) sequence which makes them preferred mRNAs for translation.

In a current system, recombinant adenovirus is generated from homologous recombination between shuttle vector and provirus vector. Due to the possible recombination between two proviral vectors, wild-type adenovirus may be generated from this process. Therefore, it is critical to isolate a single clone of virus from an individual plaque and examine its genomic structure.

Generation and propagation of the current adenovirus vectors, which are replication deficient, depend on a unique helper cell line, designated 293, which was transformed from human embryonic kidney cells by Ad5 DNA fragments and constitutively expresses E1 proteins. Since the E3 region is dispensable from the adenovirus genome, the current adenovirus vectors, with the help of 293 cells, carry foreign DNA in either the E1, the E3 or both regions. In nature, adenovirus can package approximately 105% of the wild-type genome, providing capacity for about 2 extra kb of DNA. Combined with the approximately 5.5 kb of DNA that is replaceable in the E1 and E3 regions, the maximum capacity of the current adenovirus vector is under 7.5 kb, or about 15% of the total length of the vector. More than 80% of the adenovirus viral genome remains in the vector backbone and is the source of vector-borne cytotoxicity. Also, the replication deficiency of the E1-deleted virus is incomplete. For example, leakage of viral gene expression has been observed with the currently available vectors at high multiplicities of infection (MOI).

Helper cell lines may be derived from human cells such as human embryonic kidney cells, muscle cells, hematopoietic cells or other human embryonic mesenchymal or epithelial cells. Alternatively, the helper cells may be derived from the cells of other mammalian species that are permissive for human adenovirus. Such cells include, e.g., Vero cells or other monkey embryonic mesenchymal or epithelial cells. As stated above, the preferred helper cell line is 293.

Recently, investigators disclosed improved methods for culturing 293 cells and propagating adenovirus. In one format, natural cell aggregates are grown by inoculating individual cells into 1 liter siliconized spinner flasks (Techne, Cambridge, UK) containing 100-200 ml of medium. Following stirring at 40 rpm, the cell viability is estimated with trypan blue. In another format, Fibra-Cel microcarriers (Bibby Sterlin, Stone, UK) (5 g/l) is employed as follows. A cell inoculum, resuspended in 5 ml of medium, is added to the carrier (50 ml) in a 250 ml Erlenmeyer flask and left stationary, with occasional agitation, for 1 to 4 hours. The medium is then replaced with 50 ml of fresh medium and shaking initiated. For virus production, cells are allowed to grow to about 80% confluence, after which time the medium is replaced (to 25% of the final volume) and adenovirus added at an MOI of 0.05. Cultures are left stationary overnight, following which the volume is increased to 100% and shaking commenced for another 72 hours.

Other than the requirement that the adenovirus vector be replication defective, or at least conditionally defective, the nature of the adenovirus vector is not believed to be crucial to the successful practice of the invention. The adenovirus may be of any of the 42 different known serotypes or subgroups A-F. Adenovirus type 5 of subgroup C is the preferred starting material in order to obtain the conditional replication-defective adenovirus vector for use in the present invention. This is because Adenovirus type 5 is a human adenovirus about which a great deal of biochemical and genetic information is known, and it has historically been used for most constructions employing adenovirus as a vector.

As stated above, the typical vector according to the present invention is replication defective and will not have an adenovirus E1 region. Thus, it will be most convenient to introduce the polynucleotide encoding the gene of interest at the position from which the E1-coding sequences have been removed. However, the position of insertion of the construct within the adenovirus sequences is not critical to the invention. The polynucleotide encoding the gene of interest may also be inserted in lieu of the deleted E3 region in E3 replacement vectors or in the E4 region where a helper cell line or helper virus complements the E4 defect.

Adenovirus is easy to grow and manipulate and exhibits broad host range *in vitro* and *in vivo.* This group of viruses can be obtained in high titers, e.g., 10⁹-10¹¹ plaque-forming units per ml, and they are highly infective. The life cycle of adenovirus does not require integration into the host cell genome. The foreign genes delivered by adenovirus vectors are episomal and, therefore, have low genotoxicity to host cells. No side effects have been reported in studies of vaccination with wild-type adenovirus, demonstrating their safety and therapeutic potential as in vivo gene transfer vectors.

Adenovirus vectors have been used in eukaryotic gene expression and vaccine development. Recently, animal studies suggested that recombinant adenovirus could be used for gene therapy. Studies in administering recombinant adenovirus to different tissues include trachea instillation, muscle injection, peripheral intravenous injections and stereotactic inoculation into the brain.

The retroviruses are a group of single-stranded RNA viruses characterized by an ability to convert their RNA to double-stranded DNA in infected cells by a process of reverse-transcription. The resulting DNA then stably integrates into cellular chromosomes as a provirus and directs synthesis of viral proteins. The integration results in the retention of the viral gene sequences in the recipient cell and its descendants. The retroviral genome contains three genes, gag, pol, and env that code for capsid proteins, polymerase enzyme, and envelope components, respectively. A sequence found upstream from the gag gene contains a signal for packaging of the genome into virions. Two long terminal repeat (LTR) sequences are present at the 5' and 3' ends of the viral genome. These contain strong promoter and enhancer sequences and are also required for integration in the host cell genome.

In order to construct a retroviral vector, a polynucleotide encoding a gene of interest is inserted into the viral genome in the place of certain viral sequences to produce a virus that is replication-defective. In order to produce virions, a packaging cell line containing the gag, pol, and env genes but without the LTR and packaging components is constructed. When a recombinant plasmid containing a cDNA, together with the retroviral LTR and packaging sequences is introduced into this cell line (by calcium phosphate precipitation for example), the packaging sequence allows the RNA transcript of the recombinant plasmid to be packaged into viral particles, which are then secreted into the culture media. The media containing the recombinant retroviruses is then collected, optionally concentrated, and used for gene transfer. Retroviral vectors are able to infect a broad variety of cell types. However, integration and stable expression require the division of host cells.

A novel approach designed to allow specific targeting of retrovirus vectors was recently developed based on the chemical modification of a retrovirus by the chemical addition of lactose residues to the viral envelope. This modification could permit the specific infection ofhepatocytes via sialoglycoprotein receptors.

A different approach to targeting of recombinant retroviruses was designed in which biotinylated antibodies against a retroviral envelope protein and against a specific cell receptor were used. The antibodies were coupled via the biotin components by using streptavidin. Using antibodies against major histocompatibility complex class I and class II antigens, investigators demonstrated the infection of a variety of human cells that bore those surface antigens with an ecotropic virus in vitro.

There are certain limitations to the use of retrovirus vectors in all aspects of the present invention. For example, retrovirus vectors usually integrate into random sites in the cell genome. This can lead to insertional mutagenesis through the interruption of host genes or through the insertion of viral regulatory sequences that can interfere with the function of flanking genes. Another concern with the use of defective retrovirus vectors is the potential appearance of wild-type replication-competent virus in the packaging cells. This can result from recombination events in which the intact-sequence from the recombinant virus inserts upstream from the gag, pol, env sequence integrated in the host cell genome. However, new packaging cell lines are now available that should greatly decrease the likelihood of recombination.

Lentiviruses can also be used as vectors in the present application. In addition to the long-term expression of the transgene provided by all retroviral vectors, lentiviruses present the opportunity to transduce nondividing cells and potentially achieve regulated expression. The development of lentiviral vectors requires the design of transfer vectors to ferry the transgene with efficient encapsidation of the transgene RNA and with full expression capability, and of a packaging vector to provide packaging machinery *in trans* but without helper virus production. For both vectors, a knowledge of packaging signal is required-the signal to be included in the transfer vector but excluded from the packaging vector. Exemplary human lentiviruses are human immunodeficiency virus type 1 and type 2 (HIV-1 and HIV-2). HIV-2 is likely better suited for gene transfer than HIV-1 as it is less pathogenic and thus safer during design and production; its desirable nuclear import and undesirable cell-cycle arrest functions are segregated on two separate genes.

AAV utilizes a linear, single-stranded DNA of about 4700 base pairs. Inverted terminal repeats flank the genome. Two genes are present within the genome, giving rise to a number of distinct gene products. The first, the *cap* gene, produces three different virion proteins (VP), designated VP-1, VP-2 and VP-3. The second, the *rep* gene, encodes four non-structural proteins (NS). One or more of these *rep* gene products is responsible for transactivating AAV transcription.

The three promoters in AAV are designated by their location, in map units, in the genome. These are, from left to right, p5, p19 and p40. Transcription gives rise to six transcripts, two initiated at each of three promoters, with one of each pair being spliced. The splice site, derived from map units 42-46, is the same for each transcript. The four non-structural proteins apparently are derived from the longer of the transcripts, and three virion proteins all arise from the smallest transcript.

AAV is not associated with any pathologic state in humans. Interestingly, for efficient replication, AAV requires "helping" functions from viruses such as herpes simplex virus I and II, cytomegalovirus, pseudorabies virus and, of course, adenovirus. The best characterized of the helpers is adenovirus, and many "early" functions for this virus have been shown to assist with AAV replication. Low level expression of AAV *rep* proteins is believed to hold AAV structural expression in check, and helper virus infection is thought to remove this block.

The terminal repeats of an AAV vector can be obtained by restriction endonuclease digestion of AAV or a plasmid such as psub201, which contains a modified AAV genome, or by other methods known to the skilled artisan, including but not limited to chemical or enzymatic synthesis of the terminal repeats based upon the published sequence of AAV. The ordinarily skilled artisan can determine, by well-known methods such as deletion analysis, the minimum sequence or part of the AAV ITRs which is required to allow function, *i.e*. stable and site-specific integration. The ordinarily skilled artisan also can determine which minor modifications of the sequence can be tolerated while maintaining the ability of the terminal repeats to direct stable, site-specific integration.

AAV-based vectors have proven to be safe and effective vehicle for gene delivery in *vitro,* and these vectors are now being developed and tested in pre-clinical and clinical stages for a wide range of applications in potential gene therapy, both *ex vivo* and *in vivo.* However, wide variations in AAV transduction efficiency in different cells and tissues in *vitro* as well as *in vivo* has been repeatedly observed.

AAV-mediated efficient gene transfer and expression in the lung has led to clinical trials for the treatment of cystic fibrosis. Similarly, the prospects for treatment of muscular dystrophy by AAV-mediated gene delivery of the dystrophin gene to skeletal muscle, of Parkinson's disease by tyrosine hydroxylase gene delivery to the brain, of hemophilia B by Factor IX gene delivery to the liver, and potentially of myocardial infarction by vascular endothelial growth factor gene to the heart, appear promising since AAV-mediated transgene expression in these organs has recently been shown to be highly efficient.

Other viral vectors may be employed as expression constructs in the present invention. Vectors derived from viruses such as vaccinia virus, alphaviruses, and herpesviruses may be employed. They offer several attractive features for various mammalian cells.

With the recent recognition of defective hepatitis B viruses, new insight was gained into the structure-function relationship of different viral sequences. *In vitro* studies showed that the virus could retain the ability for helper-dependent packaging and reverse transcription despite the deletion of up to 80% of its genome. This suggested that large portions of the genome could be replaced with foreign genetic material. The hepatotropism and persistence (integration) were particularly attractive properties for liver-directed gene transfer. Investigators recently introduced the chloramphenicol acetyltransferase (CAT) gene into duck hepatitis B virus genome in the place of the polymerase, surface, and pre-surface coding sequences. It was co-transfected with wild-type virus into an avian hepatoma cell line. Culture media containing high titers of the recombinant virus were used to infect primary duckling hepatocytes. Stable CAT gene expression was detected for at least 24 days after transfection.

### C. Non-viral Delivery of Expression Vectors

Several non-viral methods for the transfer of expression constructs into cultured mammalian cells also are contemplated. These include calcium phosphate precipitation, DEAE-dextran, electroporation, direct microinjection, DNA-loaded liposomes and lipofectamine-DNA complexes, cell sonication, gene bombardment using high velocity microprojectiles, and receptor-mediated transfection. Some of these techniques may be successfully adapted for *in vivo* or *ex vivo* use.

In yet another embodiment of the invention, the expression construct may simply consist of naked recombinant DNA or plasmids. Transfer of the construct may be performed by any of the methods mentioned above which physically or chemically permeabilize the cell membrane. This is particularly applicable for transfer *in vitro* but it may be applied to *in vivo* use as well. Investigators successfully injected polyomavirus DNA in the form of calcium phosphate precipitates into liver and spleen of adult and newborn mice demonstrating active viral replication and acute infection. Other investigators also demonstrated that direct intraperitoneal injection of calcium phosphate-precipitated plasmids results in expression of the transfected genes. It is envisioned that DNA encoding a polynucleotide of interest may also be transferred in a similar manner *in vivo* and express the polynucleotide.

In still another embodiment of the invention, transferring a naked DNA expression construct into cells may involve particle bombardment. This method depends on the ability to accelerate DNA-coated microprojectiles to a high velocity allowing them to pierce cell membranes and enter cells without killing them. Several devices for accelerating small particles have been developed. One such device relies on a high voltage discharge to generate an electrical current, which in turn provides the motive force. The microprojectiles used have consisted of biologically inert substances such as tungsten or gold beads.

Selected organs including the liver, skin, and muscle tissue of rats and mice have been bombarded *in vivo.* This may require surgical exposure of the tissue or cells, to eliminate any intervening tissue between the gun and the target organ. Again, DNA encoding a particular polynucleotide may be delivered via this method and still be incorporated by the present invention.

In a further embodiment of the invention, the expression construct may be entrapped in a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers. Also contemplated are lipofectamine-DNA complexes.

Liposome-mediated nucleic acid delivery and expression of foreign DNA *in vitro* has been very successful. Investigators demonstrated the feasibility of liposome-mediated delivery and expression of foreign DNA in cultured chick embryo, HeLa and hepatoma cells. Other investigators accomplished successful liposome-mediated gene transfer in rats after intravenous injection.

In certain embodiments of the invention, the liposome may be complexed with a hemagglutinating virus (HVJ). This has been shown to facilitate fusion with the cell membrane and promote cell entry of liposome-encapsulated DNA. In other embodiments, the liposome may be complexed or employed in conjunction with nuclear non-histone chromosomal proteins (HMG-1). In yet further embodiments, the liposome may be complexed or employed in conjunction with both HVJ and HMG-1. Since these expression constructs have been successfully employed in transfer and expression of polynucleotides *in vitro* and *in vivo,* then they are applicable for the present invention. Where a bacterial promoter is employed in the DNA construct, it also will be desirable to include within the liposome an appropriate bacterial polymerase.

Other expression constructs which can be employed to deliver a polynucleotide encoding a particular gene into cells are receptor-mediated delivery vehicles. These take advantage of the selective uptake of macromolecules by receptor-mediated endocytosis in almost all eukaryotic cells. Because of the cell type-specific distribution of various receptors, the delivery can be highly specific.

Receptor-mediated gene targeting vehicles generally consist of two components: a cell receptor-specific ligand and a DNA-binding agent. Several ligands have been used for receptor-mediated gene transfer. The most extensively characterized ligands are asialoorosomucoid (ASOR) and transferrin. Recently, a synthetic neoglycoprotein, which recognizes the same receptor as ASOR, has been used as a gene delivery vehicle and epidermal growth factor (EGF) has also been used to deliver genes to squamous carcinoma cells.

In other embodiments, the delivery vehicle may comprise a ligand and a liposome. For example, investigators employed lactosyl-ceramide, a galactose-terminal asialganglioside, incorporated into liposomes and observed an increase in the uptake of the insulin gene by hepatocytes. Thus, it is feasible that a polynucleotide encoding a particular gene also may be specifically delivered into a cell type such as lung, epithelial or tumor cells, by any number of receptor-ligand systems with or without liposomes. For example, epidermal growth factor (EGF) may be used as the receptor for mediated delivery of a polynucleotide encoding a gene in many tumor cells that exhibit upregulation of EGF receptor. Mannose can be used to target the mannose receptor on liver cells. Also, antibodies to CD5 (CLL), CD22 (lymphoma), CD25 (T-cell leukemia) and MAA (melanoma) can similarly be used as targeting moieties.

### D. Antisense Oligonucleotides

Antisense oligodeoxynucleotides (AS-ODNs) are single-stranded, short sequences of DNA that are complementary to specific messenger RNA (mRNA). Since AS-ODNs hybridize with the mRNA, they prevent the targeted mRNA from expressing its polypeptide product in the cell.

The oligonucleotides (or "ODNs" or "polynucleotides" or "oligos" or "oligomers" or "n-mers") of the present invention are preferably deoxyoligonucleotides (*i.e.* DNAs), or derivatives thereof; ribo-oligonucleotides (*i.e.* RNAs) or derivatives thereof; or peptide nucleic acids (PNAs) or derivatives thereof. The oligonucleotides may also comprise phosphorothioate antisense oligonucleotides.

The term "substantially complementary," when used to define either amino acid or nucleic acid sequences, means that a particular subject sequence, for example, an oligonucleotide sequence, is substantially complementary to the sequence, and thus will specifically bind to a portion of an mRNA. As such, typically the sequences will be highly complementary to the mRNA "target" sequence, and will have no more than 1, 2, 3, 4, 5, 6, 7, 8, 9, or 10 base mismatches throughout the sequence. In many instances, it may be desirable for the sequences to be exact matches, *i.e*. be completely complementary to the sequence to which the oligonucleotide specifically binds, and therefore have zero mismatches along the complementary stretch. As such, highly complementary sequences will typically bind quite specifically to the target sequence region of the mRNA and will therefore be highly efficient in reducing, and/or even inhibiting the translation of the target mRNA sequence into polypeptide product.

Substantially complementary oligonucleotide sequences will be greater than about 80 percent complementary (or '% exact-match') to the corresponding mRNA target sequence to which the oligonucleotide specifically binds, and will, more preferably be greater than about 85 percent complementary to the corresponding mRNA target sequence to which the oligonucleotide specifically binds. In certain aspects, as described above, it will be desirable to have even more substantially complementary oligonucleotide sequences for use in the practice of the invention, and in such instances, the oligonucleotide sequences will be greater than about 90 percent complementary to the corresponding mRNA target sequence to which the oligonucleotide specifically binds, and may in certain embodiments be greater than about 95 percent complementary to the corresponding mRNA target sequence to which the oligonucleotide specifically binds, and even up to and including 96%, 97%, 98%, 99%, and even 100% exact match complementary to the target mRNA to which the designed oligonucleotide specifically binds.

Percent similarity or percent complementary of any of the disclosed sequences may be determined, for example, by comparing sequence information using the GAP computer program, version 6.0, available from the University of Wisconsin Genetics Computer Group (UWGCG). The GAP program utilizes the alignment method of Needleman and Wunsch (1970). Briefly, the GAP program defines similarity as the number of aligned symbols (i.e., nucleotides or amino acids) which are similar, divided by the total number of symbols in the shorter of the two sequences. The preferred default parameters for the GAP program include: (1) a unary comparison matrix (containing a value of 1 for identities and 0 for non-identities) for nucleotides, and the weighted comparison matrix of Gribskov and Burgess (1986), (2) a penalty of 3.0 for each gap and an additional 0.10 penalty for each symbol in each gap; and (3) no penalty for end gaps.

"DNA sequence" refers to a DNA polymer, in the form of a separate fragment or as a component of a larger DNA construct. Preferably, the DNA sequences are in a quantity or concentration enabling identification, manipulation, and recovery of the sequence and its component nucleotide sequences by standard biochemical methods, for example, using a cloning vector. Such sequences are preferably provided in the form of an open reading frame uninterrupted by internal nontranslated sequences, or introns, which are typically present in eukaryotic genes. Genomic DNA containing the relevant sequences could also be used. Sequences of non-translated DNA may be present 5'or 3' from the open reading frame, where the same do not interfere with manipulation or expression of the coding regions.

"RNA sequence" refers to an RNA polymer, in the form of a separate fragment or as a component of a larger RNA construct, such as a messenger RNA (mRNA). Preferably, the RNA sequences are in a quantity or concentration enabling identification, manipulation, and recovery of the sequence and its component nucleotide sequences by standard biochemical methods, for example, using a cloning vector, or alternatively, by chemically synthesizing the RNA molecule completely or partially in vitro.

"Nucleotide sequence" refers to a heteropolymer of deoxyribonucleotides, ribonucleotides, or peptide-nucleic acid sequences that may be assembled from smaller fragments, isolated from larger fragments, or chemically synthesized *de novo* or partially synthesized by combining shorter oligonucleotide linkers, or from a series of oligonucleotides, to provide a sequence which is capable of specifically binding to an mRNA molecule and acting as an antisense construct to alter, reduce, or inhibit the transcription of the message into polypeptide, and thus, ultimately affect the concentration, amount, or activity of the final gene product *in situ, in vitro,* or *in vivo.*

The targeting of antisense oligonucleotides to bind mRNA is one mechanism to shut down protein synthesis. For example, the synthesis of polygalactauronase and the muscarine type 2 acetylcholine receptor are inhibited by antisense oligonucleotides directed to their respective mRNA sequences. Further, examples of antisense inhibition have been demonstrated with the nuclear protein cyclin, the multiple drug resistance gene (MDG1), ICAM-1, E-selectin, STK-1, striatal GABA_{A} receptor and human EGF. Antisense constructs have also been described that inhibit and can be used to treat a variety of abnormal cellular proliferations, e.g. cancer, providing proof of principle.

The oligonucleotide compounds of the invention bind to the messenger RNA having a poly A tail thereby inhibiting expression of proteins. In the specification, the letters, A, G, C, T, and U respectively indicate nucleotides in which the nucleoside is Adenosine (Ade), Guanosine (Gua), Cytidine (Cyt), Thymidine (Thy), and Uridine (Ura). As used in the specification compounds that are antisense to the DNA or mRNA sense strand are compounds which have a nucleoside sequence complementary to the sense strand. Table 3 shows the four possible sense strand nucleosides and their complements present in an antisense compound.

**TABLE 3**

| Sense | Antisense |
|---|---|
| Ade | Thy |
| Gua | Cyt |
| Cyt | Gua |
| Thy | Ade |
| Ura | Ade |

It will be understood by those skilled in the art that oligonucleotide compounds are broadly described which are capable of binding to the sense mRNA.

The antisense compounds of the present invention may also differ from native DNA in that some or all of the phosphates in the nucleotides are replaced by phosphorothioates (X=S) or methylphosphonates (X=CH₃) or other C₁₋₄ alkylphosphonates. The compounds may be further differentiated from native DNA by replacing one or both of the free hydroxy groups of the antisense molecule with C₁₋₄ alkoxy groups (R=C₁₋₄ alkoxy). As used herein, C₁₋₄ alkyl means a branched or unbranched hydrocarbon having 1 to 4 carbon-atoms.

Antisense compounds also may be substituted at the 3' and/or 5' ends by a substituted acridine derivative. As used herein, "substituted acridine" means any acridine derivative capable of intercalating nucleotide strands such as DNA. Preferred substituted acridines are 2-methoxy-6-chloro-9-pentylaminoacridine, N-(6-chloro-2-methoxyacridinyl) -O-methoxydiisopropylaminophosphinyl-3-aminopropanol, and N-(6-chloro-2-methoxyacridinyl)- O-methoxydiisopropylaminophosphinyl-5-aminopentanol. Other suitable acridine derivatives are readily apparent to persons skilled in the art. Additionally, as used herein "P(0) (0) -substituted acridine" means a phosphate covalently linked to a substitute acridine.

The oligonucleotide antisense compounds have at least 9 to about 35 or so nucleotides in length. As used herein, the term "nucleotides" includes nucleotides in which the phosphate moiety is replaced by phosphorothioate or alkylphosphonate and the nucleotides may be substituted by substituted acridines. Preferred oligonucleotide antisense compounds have at least 9 to about 25 nucleotides, while more preferred compounds have at least 9 to about 15 or so nucleotides. Compounds having fewer than 9 nucleotides are less desirable because they generally have less binding and compounds having greater than about 35 nucleotides are less desirable because their physical size and charge will attenuate the crossing of the lipophilic cell membrane. Thus, they are less likely to enter cells.

The reaction scheme involves 1H-tetrazole-catalyzed coupling of phosphoramidites to give phosphate intermediates which are reacted with sulfur in 2,6-lutidine to give phosphate compounds. Oligonucleotide compounds are prepared by treating the phosphate compounds with thiophenoxide (1:2:2 thiophenol/triethylamine/tetra-hydrofuran, room temperature, 1 h). The reaction sequence is repeated until an oligonucleotide compound of the desired length has been prepared. Such compounds are cleaved from the support by treating with ammonium hydroxide at room temperature for 1 h and then are further deprotected by heating at about 50°C overnight to yield compounds. Compounds in which at least one X is oxygen are prepared by substituting I₂-H₂O for the sulfur in 2,6-lutidine.

Antisense oligonucleotide compounds in which at least X is CH₃ or other C₁₋₄ alkyl are prepared by the following published procedure: The reaction sequence is conducted on a solid support. The reaction procedure involves phosphorylation of the 3'-hydroxyl group of a 5'-protected nucleoside using methylphosphonoditriazolide as the phosphorylating reagent followed by benzene sulfonyl-catalyzed coupling of the methylphosphonates to yield the methyl phosphonate oligonucleotide. Methylphosphonoditriazolide is prepared *in situ* from equimolar quantities of methylphosphonodichloridate, triethylamine, and triazole. Benzene sulfonyl tetrazol also was prepared in situ from pyridine, benzene sulfonic acid and triethylamine. Repeating this reaction sequence followed by cleavage from the support and deprotection yield antisense compounds.

Antisense compounds in which R is P(0) (0)-substituted acridine also are prepared by the following published procedures: These published procedures include synthesis of a nucleoside phosphoramidite-bearing acridine derivative which then is reacted with 2, 2'-dithiodiethanol attached to a support. The elongation chain then is carried out of an automatic solid-phase DNA synthesized as described above. These published procedures also include synthesis of nucleoside phosphoramidite-bearing acridine derivatives by reacting substituted 9-(3-hydroxypropyl) amino acridines with N-ethyldiisopropylamine followed by N,N-diisopropylmethylphosphonanidic chloride. Using an automated DNA synthesizer, antisense compounds in which R is P(0) (0)- substituted acridine are prepared by an extra round of synthesis using the acridinyl phosphoramidites in acetonitrile.

The inventors contemplate oligonucleotide compositions in the range of from at least 9 to about 35 or so bases in length are most preferred for the practice of the methods of the invention. In illustrative embodiments, the antisense compounds of the invention differ from native DNA by the modification of the phosphodiester backbone to extend the life of the antisense ODN, in which the phosphate substituents are replaced by phosphorothioates. Likewise, one or both ends of the oligonucleotide may be substituted by one or more acridine derivatives which intercalates nucleotide strands of DNA.

### II. Target Domains

### A. Poly A Tail

Target domains in the target nucleic acid are defined as being limited to a poly A tail and excluding AUG, 5' non-translated sequences, translation initiation factor binding sites, ribosome subunit binding sites, Shine Dalgarno sequence, 3' nontranslated sequences, poly-addition site, 3' cleavage site, coding region, intron, intron branch site, intron/exon junction, and splice sequence.

### B. Cellular

Antisense constructs may be designed to bind to the poly A tail. It is contemplated that the most effective antisense constructs consist of regions complementary to the poly A tail. The amount of poly A material included will vary depending on the particulars and circumstances. One can readily test whether too much material is included simply by testing the constructs *in vitro* to determine whether cellular function is affected or whether the expression of genes having complementary sequences is affected.

### C. Viral

Antisense molecules will also target a poly A tail within a single virus.

### III. Administration and Pharmaceutically Acceptable Carrier

In clinical applications, it will be necessary to prepare the antisense compositions of the present invention as pharmaceutical compositions, i.e. in a form appropriate for *in vivo* applications. Generally, this will entail preparing compositions that are essentially free of pyrogens, as well as other impurities that could be harmful to humans or animals.

The compositions may be administered via any suitable route, including parenterally or by injection. Solutions of the active compounds as free base or pharmacologically acceptable salts can be prepared in water suitably mixed with a surfactant, such as hydroxypropylcellulose. Dispersions also can be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial an antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the antisense constructs in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients also can be incorporated into the compositions.

The compositions of the present invention may be formulated in a neutral or salt form. Pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups also can be derived from inorganic bases such as, for example, sodium, potassium, ammonium, calcium, or ferric hydroxides, and such organic bases as isopropylamine, trimethylamine, histidine, procaine and the like.

Upon formulation, solutions will be administered in a manner compatible with the dosage formulation and in such amount as is therapeutically effective. The formulations are easily administered in a variety of dosage forms such as injectable solutions, drug release capsules and the like. For parenteral administration in an aqueous solution, for example, the solution should be suitably buffered if necessary and the liquid diluent first rendered isotonic with sufficient saline or glucose. These particular aqueous solutions are especially suitable for intramuscular, intratumor, subcutaneous and intraperitoneal administration.

In this connection, sterile aqueous media which can be employed will be known to those of skill in the art in light of the present disclosure. For example, a unit dose could be dissolved in 1 ml of isotonic NaCl solution and either added to 1000 ml of hypodermoclysis fluid or injected at the proposed site of infusion, (see for example, "Remington's Pharmaceutical Sciences" 18th Edition). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA standards.

### Example 1

Ambion's p*Silencer* 2.1-U6 hygro siRNA expression vector (Ambion Corp.) features a human U6 RNA pol III promoter, and p*Silencer* 3.1- H1 hygro contains the H1 RNA pol III promoter. These promoters are well characterized (Myslinski, E. et al. 2001 Nucleic Acids Res 29:2502-2509; Kunkel, GR and Pederson T 1989 Nucleic Acids Res **17**:7371-7379), and they provide high levels of constitutive expression across a variety of cell types. The terminator consists of a short stretch of uridines (usually 3-4 nt); this is compatible with the original siRNA design that terminates with a two uridine 3' overhang (Elbashir, SM et al. 2001 EMBO J 20:6877-6888).

The prototypical siRNA comprises two hybridized 21-mer RNA molecules with 19 complementary nucleotides and 3' terminal dinucleotide overhangs. Expression vectors with dual promoters that express the two strands of the siRNA separately can be used (Lee, NS et al. 2002 Nature Biotech 19:500-505), however, a more efficient scheme is to express a single RNA that is a 19-mer hairpin with a loop and 3' terminal uridine tract (Paddison, PJ et al. 2002 Genes and Dev 16:948-958). When expressed in mammalian cells, the hairpin siRNA can efficiently induce RNAi of the target gene (Brummelkamp, TR et al. 2002 Science 296:550-553, Sui, G. et al. 2002 Proc Natl Acad Sci USA 99:5515-5520, Paddison, PJ et al. 2002 Genes and Dev 16:948-958). For cloning into an siRNA expression vector, hairpin siRNA inserts have the advantage that only a single pair of oligonucleotides and a single ligation are needed to generate plasmid for gene silencing studies. For each target gene, complementary 55-60 mer oligonucleotides with 5' single-stranded overhangs are designed for ligation into the p*Silencer* hygro vectors. The oligonucleotides should encode 19-mer hairpin sequences specific to the mRNA target, a loop sequence separating the two complementary domains, and a poly thymidine tract to terminate transcription.

Two complementary oligonucleotides must be synthesized, annealed, and ligated into the linearized *pSilencer* vector for each siRNA target site. **Fig. 6** shows schematically how to convert siRNA target sites into oligonucleotide sequences for use in the p*silencer* vectors. The oligonucleotides encode a hairpin structure with a 19-mer stem derived from the mRNA target site. The loop of the hairpin siRNA is located close to the center of the oligonucleotides; a variety of loop sequences have been successfully used by researchers (Sui, G. et al. 2002 Proc Natl Acad Sci USA 99:5515-5520, Lee, NS et al. 2002 Nature Biotech 19:500-505; Paddsion, PJ et al. 2002 Genes and Dev 16:948-958; Brummelkamp, TR et al 2002 Science 296:550-553, Paul, CP et al. 2002 Nature Biotech 20:505-508). The loop sequence shown in **Fig. 6**, 5-UUCAAGAGA-3, is one possible sequence. Near the end of the hairpin siRNA template is a 5-6 nucleotide poly(T) tract recognized as a termination signal by RNA pol III that will terminate siRNA synthesis. The function of the 5'-GGAA-3' just downstream of the RNA pol III terminator site is not fully understood, but might be included for optimal gene silencing, especially to distinguish the antisense strand when the sense strand is a poly A sequence from the RNA pol III terminator. The 5' ends of the two oligonucleotides are noncomplementary and form the BamHI and Hind III restriction site overhangs that facilitate efficient directional cloning into the p*Silencer* vectors. Just downstream of the BamHI site, it is advantageous to have a G or an A residue because RNA pol III prefers to initiate transcription with a purine. For siRNA targets with a C or a U residue at position 1 (the first nucleotide after the AA in the RNA target sequence), an additional G (shown with an asterisk in **Fig. 6**) is added to facilitate transcription of the siRNA by RNA pol III.

### Example 2

The p*Silencer* 4.1-CMV vectors (Ambion Corp.) employ a powerful CMV promoter to drive high level expression of cloned hairpin siRNA templates in a wide variety of cell types.

Many commonly used systems for expressing siRNA in cells use an RNA polymerase III (RNA pol III) promoter such as U6 or H1. However it has recently been shown that RNA polymerase II (RNA pol II) promoters are capable of expressing high levels of functional siRNA in cells (Xia, H. et al. 2002 Nature Biotech 20:1006-1010). The p*Silencer* 4.1-CMV hygro System employs a modified Cytomegalomavirus (CMV) promoter to drive expression with RNA pol II, and includes a modified simian virus-40 (SV40) polyadenylation signal downstream of the siRNA template to terminate transcription.

The CMV promoter is considered to be a stronger promoter than the other common RNA pol II promoters used in mammalian expression vectors such as Simian virus-40 (SV40) and Rous sarcoma virus (RSV) (Foecking, MK et al. 1986 Gene 145:101-105). *In vivo,* RNA pol II is primarily responsible for transcription of mRNA within the cell. The CMV promoter has the advantage of being highly active in a broad range of cell types, and it does not interfere with other transcription events as may be the case with the RNA pol III U6 and H1 promoters in some situations.

The prototypical siRNA is comprised of two hybridized 21-mer RNA molecules with 19 complementary nucleotides and 3' terminal dinucleotide overhangs. Expression vectors with dual promoters that express the two strands of the siRNA separately can be used (Lee, NS et al. 2002 Nature Biotech 19:500-505); however, a more efficient scheme is to express a single RNA that is a 19-mer hairpin with a loop and 3' terminal overhang (Paddison, PJ et al. 2002 Genes and Dev 16:948-958). When expressed in mammalian cells, this type of hairpin siRNA or shRNA can efficiently induce RNAi of the target gene (Brummelkamp, TR et al. 2002 Science 296:550-553, Sui, G. et al. 2002 Proc Natl Acad Sci USA 99:5515-5520, Paddison, PJ et al. 2002 Genes and Dev 16:948-958). For cloning into an siRNA expression vector, hairpin siRNA inserts have the advantage that only a single pair of oligonucleotides and a single ligation are needed to generate plasmids for gene silencing studies. For each target gene, complementary 55-60 mer oligonucleotides with single-stranded overhangs encoding restriction enzyme sites are synthesized, annealed, and ligated into the p*Silencer* 4.1-CMV hygro vector. The oligonucleotide insert should encode a 19-mer hairpin sequence specific to the mRNA target, a loop sequence separating the two complementary domains, and a dinucleotide overhang that can hybridize with the RNA target.

**Fig. 7** shows an example target sequence, and how to design corresponding hairpin siRNA template oligonucleotides that will be annealed to form the DNA insert. The sense and antisense template oligonucleotides should encode a hairpin structure with a 19-mer stem and a 2 nt overhang derived from the 21 nt mRNA target site. Several different loop sequences have been successfully used in hairpin siRNA templates (Sui, G. et al. 2002 Proc Natl Acad Sci USA 99:5515-5520, Lee, NS et al. 2002 Nature Biotech 19:500-505, Paddsion, PJ et al. 2002 Genes and Dev 16:948-958, Brummelkamp, TR et al. 2002 Science 296:550-553, Paul, CP et al. 2002 Nature Biotech 20:505-508). The loop sequence shown here, 5'-TTCAAGAGA-3', is one possible sequence. The 5' ends of the two oligonucleotides form the BamH1 and Hind III restriction site overhangs that facilitate efficient directional cloning into p*silencer* 4.1-CMV hygro.

## Claims

1. An antisense nucleic acid consisting of a sequence complementary to a region in a target mRNA, wherein the region is limited to a poly A tail and excludes AUG, 5' non-translated sequences, translation initiation factor binding sites, ribosome subunit binding sites, Shine Dalgarno sequence, 3' nontranslated sequences, poly-addition site, 3' cleavage site, coding region, intron, intron branch site, intron/exon junction, and splice sequence; or an expression vector that produces the antisense nucleic acid consisting of a sequence complementary to a region in a target mRNA, wherein the region is limited to a poly A tail, and excludes AUG, 5' non-translated sequences, translation initiation factor binding sites, ribosome subunit binding sites, Shine Dalgarno sequence, 3' nontranslated sequences, poly-addition site, 3' cleavage site, coding region, intron, intron branch site, intron/exon junction, and splice sequence for use in a method of treating a tumor, whereby said antisense nucleic acid or said vector is administered into the tumor of a subject.

2. The antisense nucleic acid or the expression vector for use in a method according to claim 1, whereby the antisense nucleic acid or the expression vector is injected into the tumor.

3. A pharmaceutical composition comprising an antisense nucleic acid consisting of a sequence complementary to a region in a target mRNA, wherein the region is limited to a poly A tail and excludes AUG, 5' non-translated sequences, translation initiation factor binding sites, ribosome subunit binding sites, Shine Dalgarno sequence, 3' nontranslated sequences, poly-addition site, 3' cleavage site, coding region, intron, intron branch site, intron/exon junction, and splice sequence; or
an expression vector that produces the antisense nucleic acid consisting of a sequence complementary to a region in a target mRNA, wherein the region is limited to a poly A tail, and excludes AUG, 5' non-translated sequences, translation initiation factor binding sites, ribosome subunit binding sites, Shine Dalgarno sequence, 3' nontranslated sequences, poly-addition site, 3' cleavage site, coding region, intron, intron branch site, intron/exon junction, and splice sequence for use in a method of treating a tumor, whereby said pharmaceutical composition is administered into the tumor of a subject.

4. The pharmaceutical composition for use in a method according to claim 3, whereby said pharmaceutical composition is injected into the tumor in a subject.

5. The pharmaceutical composition for use in a method according to claim 3 or claim 4, wherein the subject is a human.

6. The antisense nucleic acid or expression vector for use in a method according to claim 1 or claim 2, which is formulated in a nanoparticle or liposome.

## Patentansprüche

1. Gegensinn-Nukleinsäure, die aus einer Sequenz besteht, die zu einer Region in einer Ziel-mRNA komplementär ist, wobei die Region auf einen Poly-A-Schwanz begrenzt ist und Folgendes ausschließt: AUG, nichttranslatierte 5'-Sequenzen, Translationsinitiationsfaktorbindungsstellen, Ribosomuntereinheitenbindungsstellen, Shine Dalgarno-Sequenz, nichttranslatierte 3'-Sequenzen, Poly-Additionsstelle, 3' Spaltungsstelle, Kodierungsregion, Intron, Intron-Verzweigungsstelle, Intron/Exon-Übergang, und Splice-Sequenz; oder
Expressionsvektor, der die Gegensinn-Nukleinsäure produziert, die aus einer Sequenz besteht, die zu einer Region in einem Ziel-mRNA komplementär ist, wobei die Region auf einen Poly-A-Schwanz begrenzt ist und Folgendes ausschließt: AUG, nichttranslatierte 5'-Sequenzen, Translationsinitiationsfaktorbindungsstellen, Ribosomuntereinheitenbindungsstellen, Shine Dalgarno-Sequenz, nichttranslatierte 3'-Sequenzen, Poly-Additionsstelle, 3' Spaltungsstelle, Kodierungsregion, Intron, Intron-Verzweigungsstelle, Intron/Exon-Übergang, und Splice-Sequenzen zur Verwendung in einem Verfahren zur Behandlung eines Tumors, wobei die Gegensinn-Nukleinsäure oder der Vektor in den Tumor eines Patienten verabreicht wird.

2. Gegensinn-Nukleinsäure oder Expressionsvektor zur Verwendung in einem Verfahren nach Anspruch 1, wobei die Gegensinn-Nukleinsäure oder der Expressionsvektor in den Tumor injiziert wird.

3. Pharmazeutische Zusammensetzung, umfassend eine Gegensinn-Nukleinsäure, die aus einer Sequenz besteht, die komplementär zu einer Region in einer Ziel-mRNA ist, wobei die Region auf einen Poly-A-Schwanz begrenzt ist und Folgendes ausschließt: AUG, nichttranslatierte 5'-Sequenzen, Translationsinitiationsfaktorbindungsstellen, Ribosomuntereinheitenbindungsstellen, Shine Dalgarno-Sequenz, nichttranslatierte 3'-Sequenzen, Poly-Additionsstelle, 3'-Spaltungsstelle, Kodierungsregion, Intron, Intron-Verzweigungsstelle, Intron/Exon-Übergang und Splice-Sequenz; oder
Expressionsvektor, der die Gegensinn-Nukleinsäure produziert, die aus einer Sequenz besteht, die zu einer Region in einem Ziel-mRNA komplementär ist, wobei die Region auf einen Poly-A-Schwanz begrenzt ist und Folgendes ausschließt: AUG, nichttranslatierte 5'-Sequenzen, Translationsinitiationsfaktorbindungsstellen, Ribosomuntereinheitenbindungsstellen, Shine Dalgarno-Sequenz, nichttranslatierte 3'-Sequenzen, Poly-Additionsstelle, 3'-Spaltungsstelle, Kodierungsregion, Intron, Intron-Verzweigungsstelle, Intron/Exon-Übergang und Splice-Sequenzen zur Verwendung in einem Verfahren zur Behandlung eines Tumors, wobei die pharmazeutische Zusammensetzung in den Tumor eines Patienten verabreicht wird.

4. Pharmazeutische Zusammensetzung zur Verwendung in einem Verfahren nach Anspruch 3, wobei die pharmazeutische Zusammensetzung in den Tumor in einem Patienten injiziert wird.

5. Pharmazeutische Zusammensetzung zur Verwendung in einem Verfahren nach Anspruch 3 oder 4, wobei der Patient ein Mensch ist.

6. Gegensinn-Nukleinsäure oder Expressionsvektor zur Verwendung in einem Verfahren nach Anspruch 1 oder 2, die bzw. der in einem Nanopartikel oder Liposom formuliert ist.

## Revendications

1. Acide nucléique antisens consistant en une séquence complémentaire à une région dans un ARNm cible, où la région est limitée à une queue poly A et exclut AUG, des séquences non traduites en 5', des sites de liaison de facteur d'initiation de la traduction, des sites de liaison de sous-unité d'un ribosome, une séquence Shine-Dalgarno, des séquences non traduites en 3', une site de polyaddition, un site de clivage en 3', une région codante, un intron, un site de branchement d'intron, une jonction intron/exon, et une séquence d'épissage ; ou
un vecteur d'expression qui produit l'acide nucléique antisens consistant en une séquence complémentaire à une région dans un ARNm cible, où la région est limitée à une queue poly A et exclut AUG, des séquences non traduites en 5', des sites de liaison de facteur d'initiation de la traduction, des sites de liaison de sous-unité d'un ribosome, une séquence Shine-Dalgarno, des séquences non traduites en 3', un site de polyaddition, un site de clivage en 3', une région codante, un intron, un site de branchement d'intron, une jonction intron/exon, et une séquence d'épissage, destiné à être utilisé dans un procédé de traitement d'une tumeur, moyennant quoi ledit acide nucléique antisens ou ledit vecteur est administré dans la tumeur d'un sujet.

2. Acide nucléique antisens ou vecteur d'expression destiné à être utilisé dans un procédé selon la revendication 1, moyennant quoi l'acide nucléique antisens ou le vecteur d'expression est injecté dans la tumeur.

3. Composition pharmaceutique comprenant un acide nucléique antisens consistant en une séquence complémentaire à une région dans un ARNm cible, où la région est limitée à une queue poly A et exclut AUG, des séquences non traduites en 5', des sites de liaison de facteur d'initiation de la traduction, des sites de liaison de sous-unité d'un ribosome, une séquence Shine-Dalgarno, des séquences non traduites en 3', une site de polyaddition, un site de clivage en 3', une région codante, un intron, un site de branchement d'intron, une jonction intron/exon, et une séquence d'épissage ; ou
un vecteur d'expression qui produit l'acide nucléique antisens consistant en une séquence complémentaire à une région dans un ARNm cible, où la région est limitée à une queue poly A et exclut AUG, des séquences non traduites en 5', des sites de liaison de facteur d'initiation de la traduction, des sites de liaison de sous-unité d'un ribosome, une séquence Shine-Dalgarno, des séquences non traduites en 3', une site de polyaddition, un site de clivage en 3', une région codante, un intron, un site de branchement d'intron, une jonction intron/exon, et une séquence d'épissage, destinée à être utilisée dans un procédé de traitement d'une tumeur, moyennant quoi ladite composition pharmaceutique est administrée dans la tumeur d'un sujet.

4. Composition pharmaceutique destinée à être utilisée dans un procédé selon la revendication 3, moyennant quoi ladite composition pharmaceutique est injectée dans la tumeur chez un sujet.

5. Composition pharmaceutique destinée à être utilisée dans un procédé selon la revendication 3 ou la revendication 4, où le sujet est un humain.

6. Acide nucléique antisens ou vecteur d'expression destiné à être utilisé dans un procédé selon la revendication 1 ou la revendication 2, qui est formulé dans une nanoparticule ou un liposome.
